# EUROPÄISCHE PATENTSCHRIFT

(11) **EP 1 432 666 B1**
(45) Veröffentlichungstag und Bekanntmachung des Hinweises auf die Patenterteilung: **03.08.2005**
(21) Anmeldenummer: 02774675.9
(22) Anmeldetag: 01.10.2002
(51) Int. Cl.: C07C 11/02, C07B 47/00, C08F 210/00

(54) **VERFAHREN ZUR HERSTELLUNG VON 1-OLEFINEN MIT PALLADIUMCARBENVERBINDUNGEN**
METHOD FOR PRODUCING 1-OLEFINS USING PALLADIUM CARBENE COMPOUNDS
PROCEDE POUR LA PRODUCTION DE 1-OLEFINES A L'AIDE DE COMPOSES CARBENE DE PALLADIUM

(30) Priorität: 06.10.2001 DE 10149348
(43) Veröffentlichungstag der Anmeldung: 30.06.2004
(73) Patentinhaber: Oxeno Olefinchemie GmbH, 45772 Marl (DE)
(72) Erfinder: BELLER, Matthias, 18211 Nienhagen (DE); JACKSTELL, Ralf, 27476 Cuxhaven (DE); KLEIN, Holger, 18069 Rostock (DE); RÖTTGER, Dirk, 45657 Recklinghausen (DE); WIESE, Klaus-Diether, 45721 Haltern (DE); MASCHMEYER, Dietrich, 45657 Recklinghausen (DE); TUCHLENSKI, Axel, 45478 Mülheim (DE); KAIZIK, Alfred, 45772 Marl (DE); SANTIAGO FERNANDEZ, Silvia, 46049 Oberhausen (DE)
(74) Vertreter: Lang, Arne, Dr.
(86) Internationale Anmeldenummer: PCT/EP2002/010971
(87) Internationale Veröffentlichungsnummer: WO 2003/031379

(56) Entgegenhaltungen:
- EP-A- 0 440 995
- WO-A-92/10450
- DE-A- 10 105 751
- US-A- 5 685 128
- US-A- 6 063 878

## Beschreibung

Die Erfindung betrifft ein Verfahren zur Herstellung von 1-Olefinen durch Telomerisation von Verbindungen mit konjugierten Doppelbindungen mit einem Telogen in Gegenwart eines Edelmetall-Telomerisationskatalysators, Hydrierung des Telomers und Spaltung des hydrierten Zwischenproduktes.

1-Olefine wie 1-Octen werden in großen Mengen in der Produktion verschiedener chemischer Produkte eingesetzt. Beispielsweise werden oberflächenaktive Stoffe, Weichmacher, Schmierstoffe und Polymere aus 1-Octen hergestellt. Ein großes Einsatzgebiet ist weiterhin die Verwendung als Comonomer in Polymeren, insbesondere in Polyethylen.

Nahezu alle zur Zeit kommerziell genutzten Verfahren zur Produktion von 1-Octen basieren auf dem Rohstoff Ethen. Ethen wird oligomerisiert und man erhält ein Produktspektrum von α-Olefinen als Hauptprodukte. Bei passender Wahl von Katalysator und Prozessbedingungen kann die Menge an 1-Octen im Produkt optimiert werden und liegt dann bei ca. 25 %. Neben diesen Verfahren, mit denen die Hauptmenge an 1-Octen produziert wird, hat die Isolierung des 1-Octens aus dem Produktspektrum der Fischer-Tropsch-Reaktion eine gewisse Bedeutung erlangt.

In der Literatur sind neben den auf Ethen basierenden Prozessen auch Verfahren bekannt, die 1,3-Butadien als Rohstoff zur Herstellung von 1-Octen einsetzen. 1-Octen ist aber nicht direkt, beispielsweise über eine Dimerisierung, aus Butadien erhältlich, sondern wird nach mehreren Prozessschritten erhalten. So beschreibt die Patentanmeldung WO 92/10450 ein Verfahren, bei dem 1,3-Butadien vorzugsweise mit Methanol oder Ethanol zu einem 2,7-Octadienylether umgesetzt wird, der nach Hydrierung zum Octylether zum 1-Octen gespalten wird. In EP-A-0 440 995 wird ein analoger Weg beschritten, die Umsetzung erfolgt im ersten Schritt aber mit einer Carbonsäure. Gemeinsam ist den Verfahren der erste Prozessschritt, den man allgemein als Telomerisation bezeichnet. Bei der Telomerisation wird allgemein ein Telogen (in EP-A-0 440 995 die Carbonsäure) mit einem Taxogen (1,3-Butadien, 2 Äquivalente) zu einem Telomer umgesetzt.

Beispiele für Telomerisationsreaktionen werden unter anderem beschrieben in E. J. Smutny, J. Am. Chem. Soc. 1967, 89, 6793; S. Takahashi, T. Shibano, N. Hagihara, Tetrahedron Lett. 1967,2451; EP-A-0 561 779, US 3 499 042, US 3 530 187, GB 1 178 812, NL 6 816 008, GB 1 248 593, US 3 670 029, US 3 670 032, US 3 769 352, US 3 887 627, GB 1 354 507, DE 20 40 708, US 4 142 060, US 4 146 738, US 4 196 135, GB 1 535 718, US 4 104 471, DE 21 61 750 und EP-A-0 218 100.

Bei den bekannten Verfahren zur Darstellung von 1-Octen auf der Basis von Butadien, wie beispielweise in WO 92/10450 oder EP-A-0 440 995 beschrieben, wird das 1-Octen durch Spaltung eines an 1-Position substituierten n-Octans erhalten. Die Selektivitäten in diesem Schritt sind dabei oftmals unbefriedigend. So wird in WO 92/10450 bei der Spaltung von 1-Methoxyoctan bei einem Umsatz von 80 % eine Selektivität zu Octenen von 66 % genannt.

Als wirksame Katalysatoren für die Telomerisation haben sich halogenfreie Palladium(0)-sowie Palladium(II)-Verbindungen erwiesen (A. Behr, in "*Aspects of Homogeneous Catalysis*"; Herausgeber R. Ugo, D. Reidel Publishing Company, Doordrecht/Boston/Lancaster, **1984**, Vol. 5, 3). Daneben wurden auch Verbindungen anderer Übergangsmetalle, wie z. B. Cobalt (R. Baker, A. Onions, R. J. Popplestone, T.N. Smith, *J. Chem. Soc., Perkin Trans. II* **1975,** 1133-1138), Rhodium, Nickel (R. Baker, D.E. Halliday, T.N. Smith, *J. Organomet. Chem.* **1972,** *35*, C61-C63; R. Baker, *Chem. Rev.* **1973,** 73, 487-530; R. Baker, A.H. Cook, T.N Smith, *J. Chem. Soc., Perkin Trans. II* **1974,** 1517-1524.) und Platin, als Katalysatoren eingesetzt. Die letztgenannten Systeme sind jedoch Palladiumkomplexen hinsichtlich Aktivität und Selektivität unterlegen.

WO 91/09822 beschreibt ein kontinuierliches Verfahren mit Palladiumacetylacetonat / 2 Äquivalenten Triphenylphosphan als Katalysator. Hier wurden Katalysatorproduktivitäten (turnover numbers) bis zu 44000 erzielt. Allerdings sind die Chemoselektivitäten bei derartigen Katalysatorumsatzzahlen für das Zielprodukt < 85 %. Die Verwendung von Palladiumkomplexen oder Palladiumsalzen in Verbindung mit Carbonsäuren zur Telomerisation von Butadien ist aus EP 0 440 995 bekannt. Die Komplexbildner sind jedoch nicht spezifiziert.

National Distillers and Chem. Corp. (US 4,642,392, US 4,831,183) beschrieben 1987 ein Verfahren zur Herstellung von Octadienylethern. Dabei wurde das Produktgemisch destillativ vom Katalysator (Palladiumacetat / 5 Äq. Triphenylphosphan) abgetrennt, der in einem hochsiedenden Lösungsmittel gelöst zurückbleibt. Der Katalysator kann bis zu zwölfmal wiederverwendet werden, wobei jeweils Phosphan ergänzt wird. Der Startansatz (Beispiel 1) lieferte den linearen Ether allerdings in nur 57 % Ausbeute (entspricht TON 2000). Das n/iso-Verhältnis der Telomere beträgt in diesem Fall nur 3.7 : 1. In US 4 831 183 wurde das Gemisch beispielsweise durch Extraktion mit Hexan von der Reaktionslösung abgetrennt. Die Telomerisation wurde dabei in Dimethylformamid oder Sulfolan mit dem Katalysatorgemisch Palladim(II)acetat / 3 Äq. Triphenylphosphinmonosulfonat durchgeführt.

Auch längerkettige primäre Alkohole wie Ethanol, Propanol und Butanol (J. Beger, H. Reichel, *J. Prakt. Chem*. **1973**, *315*, 1067) bilden mit Butadien die entsprechenden Telomere. Allerdings ist die Katalysatoraktivität der bekannten Katalysatoren hier noch geringer als in den oben genannten Fällen. So wurden unter identischen Reaktionsbedingungen [Pd(acetylacetonat)₂ / PPh₃ / Butadien / Alkohol = 1 : 2 : 2000 : 5000; 60 °C / 10 h] die Telomere von Methanol mit 88 % Ausbeute, diejenigen von Propanol mit 65 % Ausbeute und von Nonanol nur noch mit 28 % Ausbeute gebildet.

Carbonsäuren sind wie Alkohole geeignete Nucleophile in Telomerisationsreaktionen. Aus Essigsäure und Butadien erhält man in guten Ausbeuten die entsprechenden Octadienylderivate (DE 2 137 291). Das Verhältnis der linearen und verzweigten Produkte (n/iso-Verhältnis) kann über die Liganden am Palladium beeinflusst werden (D. Rose, H. Lepper, *J. Organomet. Chem.* 1973, 49, 473). Mit Triphenylphosphin als Ligand wurde ein Verhältnis 4/1 erreicht, bei Einsatz von Tris(o-methylphenyl)phosphit konnte das Verhältnis auf 17/1 gesteigert werden. Andere Carbonsäuren wie Pivalinsäure, Benzoesäure oder Methacrylsäure, aber auch Dicarbonsäuren lassen sich ebenfalls mit Butadien umsetzen

Shell Oil hat aufbauend auf die Telomerisation von konjugierten Dienen mit Carbonsäuren ein Verfahren zur Herstellung von α-Olefinen in US 5 030 792 beschrieben. Telomerisationsreaktionen, bei denen Wasser als Nucleophil eingesetzt wird, sind unter anderem von der Firma Kuraray intensiv untersucht worden (US 4 334 117, US 4 356 333, US 5 057 631). Dabei werden Phosphine, meistens wasserlösliche Phosphine, oder Phosphoniumsalze (EP 0 296 550) als Liganden eingesetzt. Der Einsatz von wasserlöslichen Diphosphinen als Ligand wird in WO 98/08794 beschrieben, DE 195 23 335 offenbart die Umsetzung von Alkadienen mit Wasser in Gegenwart von Phosphonit oder Phosphinitliganden.

GB 1 535 718 beschreibt die Telomerisation von Butadien mit Aminen, katalysiert von Palladium(0)-Komplexen. In EP 939074 und EP 773211 wird die Herstellung von Octa-2,7-diethyl-1-amin durch Telomerisation von Ammoniak und Butadien beschrieben.

Die Telomerisation von Butadien mit Nucleophilen, wie Formaldehyd, Aldehyden, Ketonen, Kohlendioxid, Schwefeldioxid, Sulfinsäuren, β-Ketoestern, β-Diketonen, Malonsäureestern, α-Formylketonen und Silanen ist ebenfalls in der Literatur beschrieben.

Zusammenfassend kann man sagen, dass die bekannten Palladiumphosphankatalysatoren für Telomerisationsreaktionen von Butadien mit Alkoholen keine befriedigenden katalytischen Wechselzahlen (Katalysatorproduktivitäten, "turnover numbers" = TON) aufweisen. Technisch angestrebte Produktivitäten von > 100.000 sind mit bekannten Systemen nicht oder kaum beschrieben. Dabei sollten gleichzeitig hohe Selektivitäten von > 95% Chemo- und Regioselektivität erreicht werden, um ein ökologisch vorteilhaftes Verfahren zu erzielen.

Es wurde gefunden, dass die Herstellung von 1-Olefinen in guten Ausbeuten und Selektivitäten gelingt, indem eine katalytische Telomerisation in Gegenwart eines Nucleophils und eines Palladiumcarbenkomplexes mit anschließender Hydrierung des Telomers und Spaltung des hydrierten Telomers durchgeführt wird.

Gegenstand der vorliegenden Erfindung ist daher ein Verfahren zur Herstellung von 1-Olefinen mit 8 bis 16 Kohlenstoffatomen durch Telomerisation eines Ausgangsolefins mit mindestens zwei konjugierten Doppelbindungen mit einem Nucleophil unter Anwesenheit eines Palladiumkatalysators, Hydrierung des so erhaltenen Telomers und anschließende Spaltung zum 1-Olefin, wobei als Palladiumkatalysator ein Palladiumcarbenkomplex verwendet wird.

Als bevorzugte Palladiumkomplexe werden solche eingesetzt, bei denen das CarbenKohlenstoffatom von zwei Stickstoffatomen gebunden ist. Dies bedeutet, dass die Carbenliganden das Strukturelement 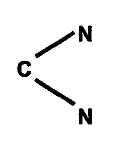 aufweisen, worin C das Carbenkohlenstoffatom darstellt, das an das Palladiumatom bindet.

Als besonders bevorzugte Palladiumcarbenkomplexe werden solche mit mindestens einem Carbenliganden der Formel I oder II 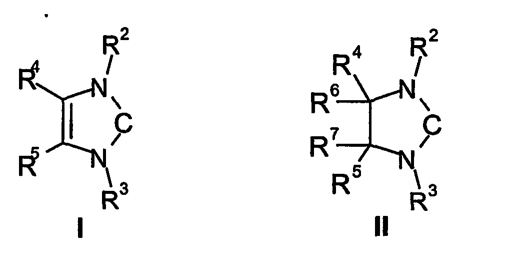 eingesetzt,
wobei R² und R³ unabhängig voneinander eine lineare, verzweigte oder cyclische C₁ bis C₂₄-Alkylgruppe oder eine C₅ bis C₁₈-Arylgruppe sind, wobei die Alkylgruppe und die Arylgruppe unabhängig voneinander die Substituenten -CN, -COOH, -COO-Alkyl-(C₁-C₈), -CO-Alkyl-(C₁-C₈), -Aryl-(C₆-C₁₈), -Alkyl-(C₁-C₂₄), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂, Ferrocenyl enthalten können,
und wobei R⁴ bis R⁷ unabhängig voneinander Wasserstoff, -CN, -COOH, -COO-Alkyl-(C₁-C₈), -CO-Alkyl-(C₁-C₈), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ oder eine lineare, verzweigte oder cyclische C₁ bis C₂₄-Alkylgruppe oder C₆ bis C₁₈-Arylgruppe ist und die Alkylgruppe und Arylgruppe unabhängig voneinander die Substituenten -CN, -COOH, -COO-Alky-(C₁-C₈), -CO-Alkyl-(C₁-C₈), -Aryl-(C₆-C₁₀), -Alkyl-(C₁-C₂₄), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂, enthalten kann, und wobei die Reste R⁴ und R⁵ auch Teil eines verbrückenden aliphatischen oder aromatischen Ringes sein können.

Die im erfindungsgemäßen Verfahren eingesetzten Nucleophile sind bevorzugt Verbindungen der Formeln III, IV und V 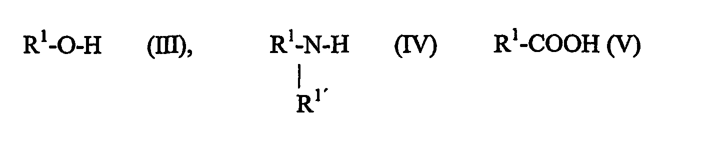 worin R¹, R^{1'} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer Carboxylgruppe oder einer C₅ bis C₁₈ Arylgruppe, wobei diese Gruppen Substituenten ausgewählt aus der Gruppe -CN, -COOH, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ enthalten können, und wobei die Reste R¹, R^{1'} über kovalente Bindungen miteinander verknüpft sein können.

Das Verfahren gemäß der Erfindung eignet sich zur Herstellung von 1-Olefinen mit 8-16 Kohlenstoffatomen, besonders bevorzugtes Produkt ist 1-Octen. Als Ausgangsolefin wird bevorzugt 1,3-Butadien oder Isopren verwendet.

Für den Telomerisationsprozess der vorliegenden Erfindung können sowohl reine Ausgangsolefine als auch Mischungen dieser Olefine mit anderen Kohlenwasserstoffen eingesetzt werden. Die weiteren Kohlenwasserstoffe sind z. B. einfach ungesättigte Verbindungen oder Alkine oder Kumulene oder Alkane. Als 1,3-Butadien enthaltende Mischungen kommen vorzugsweise Mischungen von 1,3-Butadien mit anderen C₄- oder C₅-Kohlenwasserstoffen zum Einsatz. Solche Mischungen fallen beispielsweise bei Spalt(Crack)-Prozessen zur Produktion von Ethen an, in denen Raffineriegase, Naphtha, Gasöl, LPG (liquified petroleum gas), NGL (natural gas liquid) usw. umgesetzt werden. Die bei diesen Prozessen als Nebenprodukt anfallenden C₄-Schnitte enthalten je nach Spalt-Verfahren unterschiedliche Mengen an 1,3-Butadien. Typische 1,3-Butadienkonzentrationen im C₄-Schnitt, wie sie aus einem Naphtha-Steamcracker erhalten werden, liegen bei 20 - 70 % 1,3-Butadien.

Die C₄-Komponenten n-Butan, i-Butan, 1-Buten, cis-2-Buten, trans-2-Buten und i-Buten, die ebenfalls in diesen Schnitten enthalten sind, stören die Umsetzung im Telomerisationsschritt nicht oder nicht wesentlich. Diene mit kumulierten Doppelbindungen (1,2-Butadien, Allen usw.) und Alkine, insbesondere Vinylacetylen, können hingegen als Moderatoren in der Telomerisationsreaktion wirken. Es ist daher vorteilhaft, die C₄-Alkine und gegebenenfalls das 1,2-Butadien vorher zu entfernen (DE 195 23 335). Dies kann, falls möglich, über physikalische Verfahren wie Destillation oder Extraktion erfolgen. Auf chemischem Wege können die Alkine über Selektivhydrierungen zu Alkenen oder Alkanen und die kumulierten Diene zu Monoenen reduziert werden. Verfahren für derartige Hydrierungen sind Stand der Technik und zum Beispiel in WO 98/12160, EP-A-0 273 900, DE-A-37 44 086 oder US 4 704 492 beschrieben.

Als Nucleophile (Telogene) können alle Verbindungen eingesetzt werden, die den allgemeinen Formeln III bis V genügen. Speziell sind dies:
- Wasser, Ammoniak
- Monoalkohole und Phenole wie zum Beispiel Methanol, Ethanol, n-Propanol, Isopropanol, Allylalkohol, n-Butanol, i-Butanol, Octanol, 2-Ethylhexanol, Isononanol, Benzylalkohol, Cyclohexanol, Cyclopentanol oder 2,7-Octadien-1-ol, Phenol
- Dialkohole wie zum Beispiel Ethylenglykol, 1,2-Propandiol, 1,3-Propandiol, 1,4-Butandiol, 1,2-Butandiol, 2,3-Butandiol und 1,3-Butandiol
- Hydroxyverbindungen wie zum Beispiel α-Hydroxyessigsäureester
- primäre Amine wie zum Beispiel Methylamin, Ethylamin, Propylamin, Butylamin, Octylamin, 2,7-Octadienylamin, Dodecylamin, Ethylendiamin oder Hexamethylendiamin
- sekundäre Amine wie Dimethylamin, Diethylamin, N-Methylanilin, Bis(2,7-Octadienyl)amin, Dicyclohexylamin, Methylcyclohexylamin, Pyrrolidin, Piperidin, Morpholin, Piperazin oder Hexamethylenimin
- Carbonsäuren wie Ameisensäure, Essigsäure, Propansäure, Butensäure, Isobutensäure, Benzoesäure, 1,2 Benzoldicarbonsäure (Phthalsäure).

Besonders bevorzugte Nucleophile sind Methanol, Ethanol, 2-Ethlyhexanol, Octanol, Octenol, Octadienol, Isopropanol, n-Propanol, Isobutanol, n-Butanol, Isononanol, Ameisensäure, Essigsäure, Propionsäure, n-Butansäure, iso-Butansäure, Benzoesäure, Phthalsäure und/oder Wasser.

Nucleophile, die selbst über eine Telomerisationsreaktion erhalten werden können, können direkt eingesetzt oder aber in situ gebildet werden. So kann beispielsweise 2,7-Octadien-1-ol aus Wasser und Butadien in Anwesenheit des Telomerisationskatalysators in situ gebildet werden, 2,7-Octadienylamin aus Ammoniak und 1,3-Butadien usw.

Für das Verhältnis von Nucleophil zum Ausgangsolefin mit mindestens zwei konjugierten Doppelbindungen in der Telomerisationsreaktion ist die Anzahl der aktiven Wasserstoffatome im Telogen zu berücksichtigen. So hat beispielsweise Methanol ein aktives Wasserstoffatom, Ethylenglykol hat zwei, Methylamin hat zwei usw.

Pro Mol aktivem Wasserstoffatom des Nucleophils, das mit dem Ausgangsolefin reagieren kann, werden bevorzugt 0,001 Mol bis 10 Mol Ausgangsolefin in der Telomerisationsreaktion eingesetzt. Bei einer Reaktionsführung mit einer flüssigen Phase wird ein Verhältnis von 0,1 Mol bis 2 Mol Ausgangsolefin pro Mol aktivem Wasserstoff besonders bevorzugt.

Als Lösemittel für die Telomerisationsreaktion findet im allgemeinen das eingesetzte Nucleophil Verwendung, wenn es bei Reaktionsbedingungen als Flüssigkeit vorliegt. Es können jedoch auch andere Lösemittel eingesetzt werden. Die eingesetzten Lösemittel sollten dabei weitgehend inert sein. Bevorzugt wird der Zusatz von Lösemitteln bei Einsatz von Nucleophilen, die unter Reaktionsbedingungen als Feststoffe vorliegen oder bei Produkten, die unter den Reaktionsbedingungen als Feststoffe anfallen würden. Geeignete Lösemittel sind unter anderem aliphatische, cycloaliphatische und aromatische Kohlenwasserstoffe wie zum Beispiel C₃-C₂₀-Alkane, Mischungen niederer Alkane (C₃-C₂₀), Cyclohexan, Cyclooctan, Ethylcyclohexan, Alkene und Polyene, Vinylcyclohexen, 1,3,7-Octatrien, die C₄-Kohlenwasserstoffe aus Crack-C₄-Schnitten, Benzol, Toluol und Xylol; polare Lösemittel wie zum Beispiel tertiäre und sekundäre Alkohole, Amide wie zum Beispiel Acetamid, Dimethylacetamid und Dimethylformamid, Nitrile wie zum Beispiel Acetonitril und Benzonitril, Ketone wie zum Beispiel Aceton, Methylisobutylketon und Diethylketon; Carbonsäureester wie zum Beispiel Essigsäureethylester, Ether wie beispielsweise Dipropylether, Diethylether, Dimethylether, Methyloctylether, 3-Methoxyoctan, Dioxan, Tetrahydrofuran, Anisol, Alkyl- und Arylether von Ethylenglycol, Diethylenglycol und Polyethylenglycol und andere polare Lösemittel wie zum Beispiel Sulfolan, Dimethylsulfoxid, Ethylencarbonat, Propylencarbonat und Wasser. Auch Ionische Flüssigkeiten, beispielsweise Imidazolium oder Pyridiniumsalze, können als Lösemittel eingesetzt werden.

Die Lösemittel kommen allein oder als Mischungen verschiedener Lösemittel zum Einsatz.

Die Temperatur, bei der die Telomerisationsreaktion ausgeführt wird, liegt zwischen 10 und 180 °C, bevorzugt zwischen 30 und 120 °C, besonders bevorzugt zwischen 40 und 100 °C. Der Reaktionsdruck beträgt 1 bis 300 bar, bevorzugt 1 bis 120 bar, besonders bevorzugt 1 bis 64 bar und ganz besonders bevorzugt 1 bis 20 bar.

Essentiell für das erfindungsgemäße Verfahren ist, dass die Telomerisationsreaktion mit Katalysatoren auf Basis von Palladium-Komplexen mit Carbenliganden durchgeführt wird.

Die Palladiumcarbenkomplexe können als solche in die Telomerisationsreaktion eingesetzt oder in situ während dieser Reaktion erzeugt werden.

Beispiele für Carbenliganden, die den allgemeinen Formeln **I** oder **II** entsprechen, und Komplexe, die derartige Liganden enthalten sind in der Fachliteratur bereits beschrieben (W. A. Herrmann, C. Köcher, *Angew. Chem.* **1997,** *109*, 2257; Angew. Chem. Int. Ed. Engl. **1997**, *36*, 2162; V.P.W. Böhm, C.W.K. Gstöttmayr, T. Weskamp, W.A. Herrmann, *J. Organomet. Chem*. **2000**, *595*, 186; DE 44 47 066).

Im Rahmen dieser Erfindung werden unter Carbenliganden sowohl freie Carbene, die als Ligand fungieren können, als auch an Palladium koordinierte Carbene verstanden.
Es ist möglich, gleichzeitig verschiedene Carbenliganden im erfindungsgemäßen Verfahren einzusetzen.

Das Katalysatormetall Palladium, aus dem die sich unter Reaktionsbedingungen die aktiven Katalysatoren bilden, kann auf verschiedene Weisen in den Prozess eingebracht werden.
a) Als Palladium-Carbenkomplex, wobei das Palladium bevorzugt in den Oxidationsstufen (II) oder (0) vorliegt.
b) In Form von Vorstufen, aus denen in situ die Katalysatoren gebildet werden.

### Zu a)

Beispiele sind Palladium(0)carben-olefin-Komplexe, Palladiumcarbenphosphinkomplexe, Palladium(0)dicarbenkomplexe und Palladium(II)dicarbenkomplexe, Palladium(0)carben-1,6-dien-Komplexe. Als 1,6-Dien können beispielsweise Diallylamin, 1,1'-Divinyltetramethyldisiloxan, 2,7-Octadienylether oder 2,7-Octadienylamine fungieren. Konkrete Beispiele für geeignete Palladium-carben-komplexe sind in der folgenden Tabelle wiedergegeben.

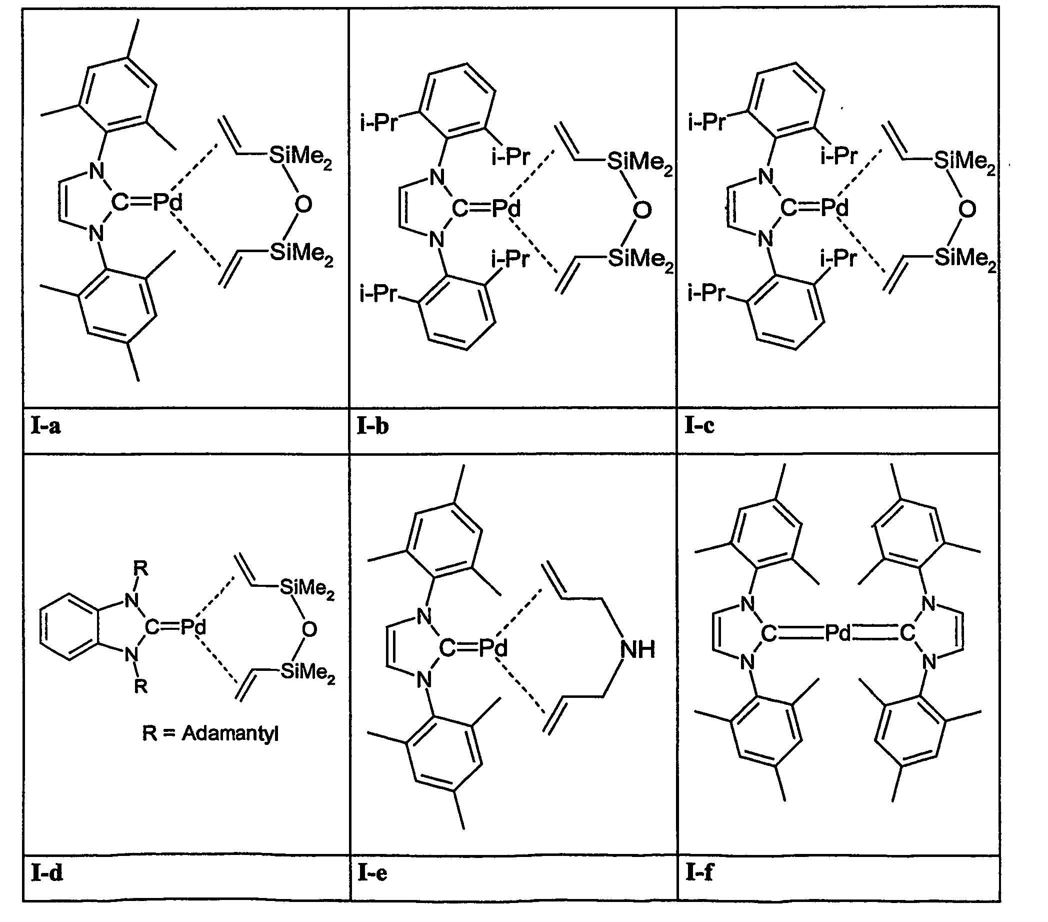

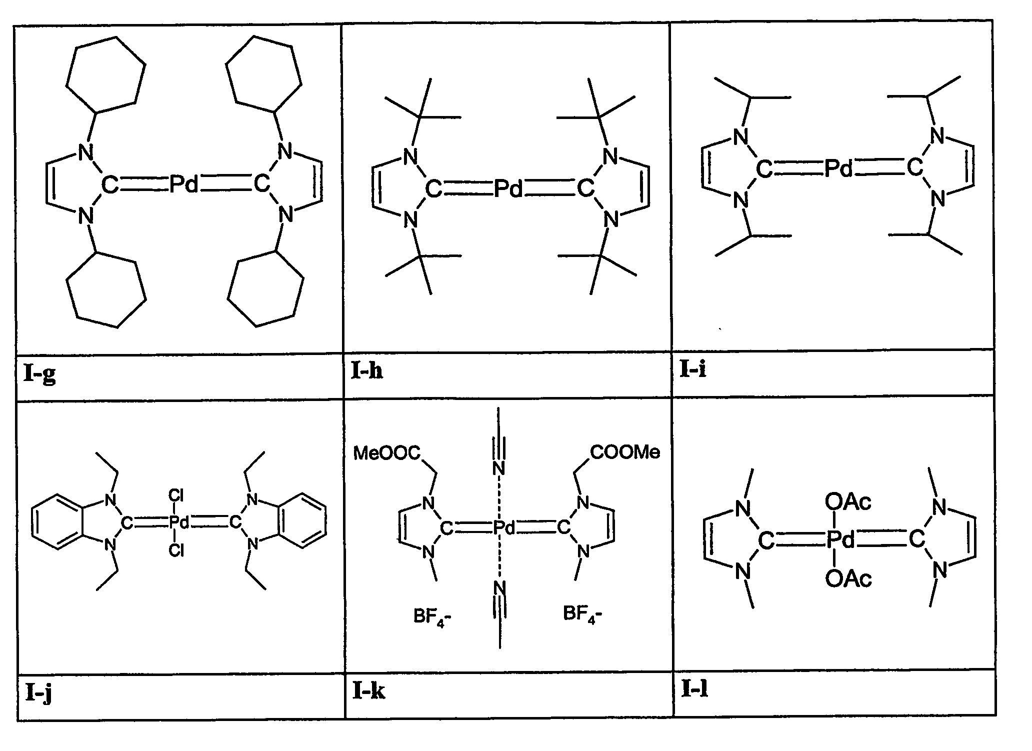

Die im erfindungsgemäßen Verfahren einzusetzenden Carbenkomplexe des Palladiums können auf verschiedenste Weisen dargestellt werden. Ein einfacher Weg ist beispielsweise die Anlagerung von Carbenliganden oder der Austausch von Liganden an Palladiumkomplexen durch Carbenliganden. So sind beispielsweise die Komplexe **I-f** bis **I-i** durch Austausch der Phosphorliganden des Komplexes Bis(tri-o-tolylphosphin)palladium(0) erhältlich (T. Weskamp, W.A. Herrmann, *J. Organomet. Chem.* **2000**, *595*, 186).

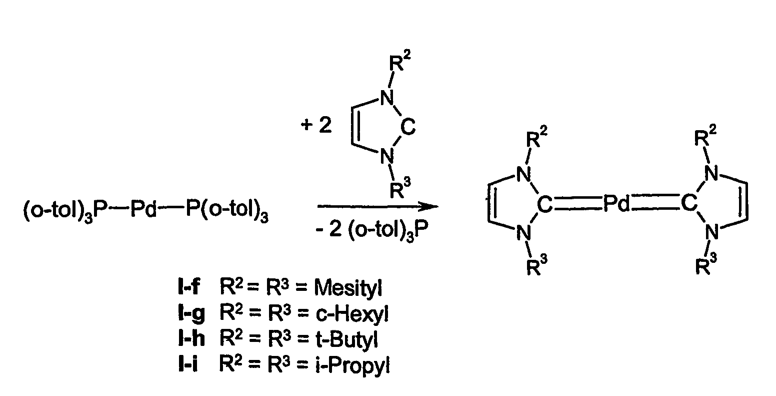

### Zu b)

Als Vorstufen der Palladiumkatalysatoren können Palladiumsalze, wie beispielsweise Palladium(II)acetat, Palladium(II)chlorid, Palladium(II)bromid, Lithiumtetrachloropalladat, Palladium(II)acetylacetonat, Palladium(0)-dibenzylidenaceton-Komplexe, Palladium(II)propionat, Palladium(II)chloridbisacetonitril, Palladium(II)-bistriphenylphosphandichlorid, Palladium(II)chloridbisbenzonitril, Bis(tri-o-tolylphosphin)palladium(0) und weitere Palladium(0)- und Palladium(II)-Komplexe eingesetzt werden.

Die Carbene werden in Form freier Carbene oder als Metallkomplexe eingesetzt oder in situ aus Carbenvorstufen erzeugt.

Als Carbenvorstufen der Carbene nach den allgemeinen Formeln **I** und **II** eignen sich beispielsweise Salze der Carbene gemäß den allgemeinen Formeln **VI** und **VII,** 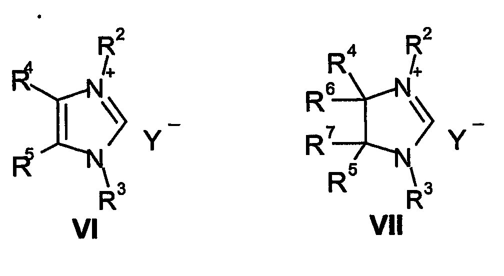 wobei R², R³, R⁴, R⁵, R⁶, R⁷ dieselbe Bedeutung haben wie in Formeln I und II und Y für eine einfach geladene anionische Gruppe oder entsprechend der Stöchiometrie anteilig für eine mehrfach geladene anionische Gruppe steht.

Beispiele für Y sind Halogenide, Hydrogensulfat, Sulfat, Alkylsulfate, Arylsulfate, Borate, Hydrogencarbonat, Carbonat, Alkylcarboxylate, Phosphate oder Arylcarboxylate.

Aus den Salzen der Carbene können die entsprechenden Carbene beispielsweise durch Umsetzung mit einer Base freigesetzt werden.

Die Konzentration des Katalysators, formal angegeben in ppm (Masse) an Palladium-Metall bezogen auf die Gesamtmasse, beträgt 0.01 ppm bis 1000 ppm, bevorzugt 0.5 bis 100 ppm, besonders bevorzugt 1 bis 50 ppm.
Das Verhältnis [Mol/Mol] von Carben zu Pd beträgt 0.01:1 bis 250:1, bevorzugt 1:1 bis 100:1, besonders bevorzugt 1:1 bis 50:1.

Es ist möglich, den Telomerisationsprozeß in Gegenwart von weiteren Liganden durchzuführen. Prinzipiell sind hierzu alle Liganden geeignet, die die Reaktionsgeschwindigkeit erhöhen, die Selektivität der Bildung des Telomers verbessern, die Katalysatorstandzeit verlängern usw. Beispiele für geeignete weitere Liganden sind Verbindungen mit einem oder mehreren trivalenten Phosphor-, Arsen-, Antimon- oder Stickstoffatomen.

### Beispiele für Phosphorliganden sind:

Phosphine wie zum Beispiel Triphenylphosphin, Tris(p-tolyl)phosphin, Tris(m-tolyl)phosphin,
Tris(o-tolyl)phosphin, Tris(p-methoxyphenyl)phosphin, Tris(p-dimethylaminophenyl)phosphin, Tricyclohexylphosphin, Tricyclopentylphosphin, Triethylphosphin, Tris(1-naphthyl)phosphin, Tribenzylphosphin, Tri-n-butylphosphin, Tri-tert.-butylphosphin, Tris(3-sulfonato-phenyl)phosphin (Metallsalz), Bis(3-sulfonato-phenyl)phenylphosphin (Metallsalz), (3-sulfonatophenyl)diphenylphosphin (Metallsalz),
Phosphite wie zum Beispiel Trimethylphosphit, Triethylphosphit, Tri-n-propylphosphit, Tri-i-propylphosphit, Tri-n-butylphosphit, Tri-i-butylphosphit, Tri-tert.-butylphosphit, Tris(2-ethylhexyl)phosphit, Triphenylphosphit, Tris(2,4-di-tert.-butylphenyl)phosphit, Tris(2-tert.-butyl-4-methoxyphenyl)phosphit, Tris(2-tert.-butyl-4-methylphenyl)phosphit, Tris(p-kresyl)phosphit, Phosphonite wie zum Beispiel Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind,
Phosphinite wie zum Beispiel Diphenyl(phenoxy)phosphin und dessen Derivate, in denen die Wasserstoffatome ganz oder teilweise durch Alkyl- und/oder Arylreste oder Halogenatome ersetzt sind, Diphenyl(methoxy)phosphin, Diphenyl(ethoxy)phosphin usw.

Im Rahmen dieser Erfindung werden auch Phosphoniumsalze als weitere Liganden verstanden. Beispiele für geeignete Phosphoniumsalze und ihren Einsatz in der Telomerisation finden sich unter anderem in EP-A-0 296 550.

Das Verhältnis von weiteren Liganden zu Palladium kann 0,1/1 bis 500/1, bevorzugt 0,5/1 bis 50/1, besonders bevorzugt 1/1 bis 20/1 [Mol/Mol] betragen. Der weitere Ligand kann der Reaktion in Substanz, gelöst oder in Form von Metallkomplexen zugeführt werden. Zusätzlicher Ligand kann der Reaktion zu jedem Zeitpunkt und an beliebiger Stelle im Reaktor in Substanz, als Lösung oder in Form eines Metallkomplexes zugeführt werden.

Aufgrund der Katalysatoraktivitäten und -stabilitäten ist es bei dem erfindungsgemäßen Verfahren möglich, kleine Mengen an Katalysator zu verwenden. Neben einer Verfahrensführung, bei der der Katalysator wiederverwendet wird, wird so auch die Option eröffnet, den Katalysator nicht zu recyceln. Beide Varianten sind in der Patentliteratur bereits beschrieben (WO 90/13531, US 5254782, US 4642392).

Oftmals ist es vorteilhaft, die Telomerisationsreaktion in Gegenwart von Basen durchzuführen. Bevorzugt werden basische Komponenten mit einem pK_{b}-Wert kleiner 7, insbesondere Verbindungen ausgewählt aus der Gruppe Amine, Alkalimetallsalze oder Erdalkalimetallsalze eingesetzt.
Als basische Komponente sind beispielsweise geeignet Amine wie Trialkylamine, die alicyclisch oder/und offenkettig sein können, Amide, Alkali- oder/und Erdalkalisalze aliphatischer oder/und aromatischer Carbonsäuren, wie Acetate, Propionate, Benzoate bzw. entsprechende Carbonate, Hydrogencarbonate, Kohlendioxid, Alkoholate von Alkali- und/oder Erdalkalielementen, Phosphate, Hydrogenphosphate oder/und Hydroxide bevorzugt von Lithium, Natrium, Kalium, Calcium, Magnesium, Cäsium, Ammonium- und Phosphoniumverbindungen. Bevorzugt sind als Zusatz Hydroxide der Alkali- und Erdalkalielemente und Metallsalze der Nucleophile nach den allgemeinen Formeln **III - V.**

Im Allgemeinen wird die basische Komponente zwischen 0.00001 Mol-% und 10 Mol-% (bezogen auf das Ausgangsolefin), bevorzugt zwischen 0.0001 Mol-% und 5 Mol-% und ganz besonders bevorzugt zwischen 0.001 Mol-% und 1 Mol-% eingesetzt.

Zusätzliche Base kann der Reaktion zu jedem Zeitpunkt und an beliebiger Stelle im Reaktor in Substanz oder in Lösung zugeführt werden.

In dem erfindungsgemäßen Verfahren beträgt das Verhältnis [Mol/Mol] zwischen eingesetztem Ausgangsolefin und Nucleophil 1:100 bis 100:1, bevorzugt 1:50 bis 10:1, besonders bevorzugt 1:10 bis 2:1.

Die Telomerisationsreaktion des erfindungsgemäßen Verfahrens kann kontinuierlich oder diskontinuierlich betrieben werden und ist nicht auf den Einsatz bestimmter Reaktortypen begrenzt. Beispiele für Reaktoren, in denen die Reaktion durchgeführt werden kann, sind Rührkessekeaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Bei dem erfindungsgemäßen Verfahren werden Carbenliganden bei Telomerisationsreaktionen eingesetzt. Überraschenderweise übertreffen diese Katalysatoren die bekannten Palladium-Phosphan-Katalysatoren sowohl in der Selektivität als auch Produktivität. Bei dem erfindungsgemäßen Verfahren können beispielsweise bei der Telomerisation von Butadien mit Alkoholen problemlos Turnover-Werte der Katalysatoren (Katalysatorproduktivitäten) in der Größenordnung von 100.000 und mehr realisiert werden.

Des weiteren ist es möglich, die Telomerisation in mehrphasigen Systemen durchzuführen (zum Beispiel heterogenkatalysiert oder in Anwesenheit von zwei flüssigen Phasen, von denen eine den Katalysator enthält). Die Konzentrationsbereiche in denen der Katalysator eingesetzt wird, können sich dabei verschieben. Bei der Telomerisation in mehreren flüssigen Phasen ist es besonders vorteilhaft, wenn Katalysator und Produkt in unterschiedlichen Phasen vorliegen, da dann der Katalysator einfach über eine Phasentrennung abgetrennt werden kann. Oftmals bildet dabei Wasser eine der flüssigen Phasen aus. Es werden beispielsweise aber auch perfluorierte Kohlenwasserstoffe, ionische Flüssigkeiten und überkritisches Kohlendioxid eingesetzt (zu ionischen Flüssigkeiten vgl. P. Wasserscheid, W. Keim, Angew. Chem., Int. Ed. 2000, 39, 3772 - 3789). Die Telomerisation von Butadien mit Wasser in ionischen Flüssigkeiten beschreiben J. E. L. Dullius, P. A. Z. Suarez, S. Einloft, R. F. de Souza, J.

Dupont, J. Fischer, A. D. Cian, Organometallics 1999, 17, 997 - 1000. Einen Überblick über Wasser als Trägerphase für den Katalysator findet man beispielsweise in B. Cornils, W. A. Herrmann (Eds.) "Aqueous-Phase Organometallic Catalysis", Wiley-VCH, Weinheim, New-York, Chichester, Brisbane, Singapore, Toronto, 1998, Seiten 442-446. Besonders vorteilhaft ist es bei Verfahren mit mehreren flüssigen Phasen ein Telogen einzusetzen, das zusammen mit dem Katalysator in einer Phase vorliegt, die Produkte sich aber hauptsächlich in einer zweiten Phase befinden.
Für die Durchführung des Telomerisationsschritts kann der Zusatz anderer Hilfsstoffe Vorteile bringen, beispielsweise der Einsatz von Inhibitoren, die die Polymerisation des Butadiens unterdrücken. Derartige Inhibitoren sind normalerweise auch im kommerziellen, (stabilisierten) reinen 1,3-Butadien enthalten. Ein Standardstabilisator ist beispielsweise tert.-Butylbrenzkatechol.

Die Telomerisationsreaktion wird bevorzugt nicht zu einem vollständigen Umsatz des Ausgangsolefins durchgeführt. Es ist häufig vorteilhaft, den Umsatz auf maximal 95, bevorzugt 88 % zu begrenzen.

Der Telomerisationskatalysator kann nach der Telomerisationsreaktion zurückgewonnen und ganz oder teilweise für weitere Telomerisationsreaktionen eingesetzt werden (vgl. EP-A-0 218 100). Die Abtrennung des Katalysators kann beispielsweise über eine Destillation, Extraktion, Fällung oder Adsorption erfolgen. Liegt der Katalysator ganz oder teilweise in einer zweiten Phase vor, kann die Trennung einfach durch Trennung der Phasen erfolgen.

Es ist auch möglich, dass der Katalysator vor der Abtrennung oder während der Abtrennung modifiziert wird. Dies gilt analog für die vollständige oder teilweise Rückführung in den Prozess, der ebenfalls eine Modifikation des Katalysators vorgeschaltet werden kann. Beispielsweise wird in US 4 146 738 ein Verfahren beschrieben, bei dem vor der Katalysatorabtrennung der Katalysator durch Hilfsstoffe stabilisiert wird. Nach der Trennung von den anderen Produkten erfolgt eine Aktivierung und Rückführung in den Prozess.

Alternativ kann der Katalysator nach der Reaktion auch anderweitig aufgearbeitet werden (vgl. WO 90/13531, US 5 254 782).

Wird das eingesetzte Telogen nicht vollständig umgesetzt, wird das überschüssige Telogen bevorzugt vom Austrag der Telomerisationsreaktion abgetrennt und ganz oder teilweise in diese zurückgeführt.

Dient das erfindungsgemäße Verfahren zur Herstellung von 1-Octen durch Telomerisation von 1,3-Butadien, so werden als Nebenprodukte hauptsächlich an der 3-Position substituiertes 1,7-Octadien, 1,3,7-Octatrien, 4-Vinylcyclohexen und weitere C₈-Olefine erhalten. Hinzu kommen geringe Mengen an höhersiedenden Komponenten. Für das weitere Verfahren kann es vorteilhaft sein, die Nebenprodukte ganz oder teilweise vom Produkt der Telomerisationsreaktion abzutrennen. Grundsätzlich können alle Verfahren oder Kombinationen von Verfahren zur Anwendung kommen, mit denen das Telomer aus dem Produktgemisch abgetrennt werden kann. Bevorzugte Trenntechnik ist die Destillation. Für die destillative Trennung können alle verfügbaren Techniken eingesetzt werden, beispielsweise Bodenkolonnen, Kolonnen mit Packungen, Trennwandkolonnen, Extraktivdestillation, Dünnschichtverdampfer und Fallfilmverdampfer. Die destillative Trennung kann in einem oder in mehreren Schritten erfolgen und ist abhängig von den Siedepunkten der im Produktgemisch enthaltenen Komponenten. Werden butadienhaltige Mischungen von C₄-Kohlenwasserstoffen als Edukte eingesetzt, haben die verbleibenden C₄-Kohlenwasserstoffe den niedrigsten Siedepunkt und können daher einfach über Kopf abgetrennt werden.

Wenn in den verbleibenden C₄-Kohlenwasserstoffen Isobuten enthalten ist und als Telogen Alkohole eingesetzt werden, ergibt sich zudem die Möglichkeit, überschüssigen Alkohol zusammen mit den C₄-Kohlenwasserstoffen abzutrennen und in anderen Prozessen weiter umzusetzen. Ist beispielsweise Isobuten in den C₄-Kohlenwasserstoffen enthalten und es wird Methanol als Telogen eingesetzt, so können nach der Telomerisation verbleibende C₄-Kohlenwasserstoffe zusammen mit überschüssigem Methanol abgetrennt und gemeinsam in eine MTBE Synthese eingespeist werden. Dabei kann es gegebenenfalls vorteilhaft sein, Reste aus nicht eingesetztem Dien vorher selektiv zum Olefin zu hydrieren.

Es kann weiterhin von Vorteil sein, andere Komponenten des Austrags der Telomerisationsreaktion zu isolieren und gegebenenfalls in den Prozess zurückzuführen oder separat zu verwerten. Für die hierzu eingesetzten Techniken gilt dasselbe wie bei der o. g.

Isolierung. Als zu isolierende Komponenten eignen sich z. B. das eingesetzte Telogen, überschüssiges 1,3-Butadien, das an 3-Position substituierte 1,7-Octadien, 1,3,7-Octatrien, 4-Vinylcyclohexen, die eingesetzte Base beziehungsweise die eingesetzten Basen und gegebenenfalls eingesetzte Lösemittel.

Der Austrag der Telomerisationsreaktion wird anschließend, ggf. zusammen mit den Nebenprodukten mit Wasserstoff oder Wasserstoff enthaltenden Gasen hydriert.

Dabei werden die ungesättigten olefinischen Doppelbindungen zu Einfachbindungen umgesetzt.

Bei Einsatz von Alkoholen als Nucleophil (Telogen) und 1,3-Butadien als Taxogen werden beispielsweise in der Telomerisation als Hauptprodukt 2,7-Octadienylether gebildet, die in der Hydrierung zu n-Octylethern umgesetzt werden. Analog erhält man aus 2,7-Octadienylestern n-Octylester, aus 2,7-Octadienylamin-n-Octylamine.

Die Hydrierung kann als Flüssig- und/oder Gasphasenhydrierung oder in Kombination dieser Techniken erfolgen und sie kann in einem oder mehreren Schritten erfolgen wie beispielsweise in einer Vor- und einer Endhydrierung.

Als Reaktoren für die Hydrierung können die bekannten Standards für Hydrierungen eingesetzt werden, beispielsweise Tricklebed-Reaktoren. Die bei der Reaktion entstehende Reaktionswärme wird nach bekannten Verfahren abgeführt, beispielsweise durch interne oder externe Kühler. Konkret kann dies den Einsatz von Rohrbündelreaktoren, Kühlfingern, Kühlschlangen oder -platten oder die Kühlung eines Rückführungsstroms (Reaktoren mit Kreislauf, Recycling), bedeuten.
Die Hydrierung wird katalysiert durchgeführt. Es können dabei sowohl homogene als auch heterogene Katalysatoren eingesetzt werden. Bevorzugt werden heterogene Katalysatoren, eingesetzt, die mindestens ein Metall der Gruppen 6-11 des Periodensystems der Elemente enthalten.
Besonders bevorzugt enthalten die Katalysatoren für diese Hydrierung Kupfer, Chrom und mindestens eines der Metalle der 8.-10. Gruppe des Periodensystems.

Bei Einsatz von homogenen Katalysatoren werden neben dem Katalysatormetall zusätzliche Liganden eingesetzt. Geeignete Liganden sind beispielsweise Verbindungen des dreiwertigen Phosphors (zum Beispiel Phosphine oder Phosphite), Verbindungen des dreiwertigen Arsens oder Antimons, Stickstoffverbindungen (zum Beispiel Amine, Pyridine, Nitrile), Halogenide, Kohlenmonoxid, Cyanid und Carbene.

Bei heterogenen Katalysatoren können die oben genannten Metalle mit anderen Metallen oder Moderatoren modifiziert sein. So werden zum Beispiel heterogene Palladiumkatalysatoren oftmals durch Zusatz von Schwefel oder Kohlenmonoxid in ihrer Aktivität und Selektivität modifiziert. Kupfer-Katalysatoren wird oftmals ein Anteil an Chrom zugesetzt.

Der Einsatz von geträgerten Katalysatoren ist in der Regel vorteilhaft, da geringere Metallmengen benötigt werden und über die Beschaffenheit des Trägers zusätzlich die Eigenschaften des Katalysators beeinflusst werden können. Als Trägermaterialien haben sich beispielsweise Aktivkohle, Aluminiumoxide, Siliziumdioxide, Silizium-Aluminium-Oxide, Bariumcarbonat, Bariumsulfat oder Kieselgur bewährt.

In einer bevorzugten Ausführungsform der vorliegenden Erfindung wird zur Hydrierung des Telomers und gegebenenfalls nicht umgesetzten Ausgangsolefins ein heterogener Palladium, Nickel oder Platin enthaltender Katalysator eingesetzt.

Besonders bevorzugt werden geträgerte heterogene Palladium- oder Platinkatalysatoren eingesetzt, wobei der Metallgehalt bevorzugt zwischen 0,01 - 10, bevorzugt zwischen 0,1-1 % liegt.

Die Hydrierungen werden bei Temperaturen von 0 bis 400 °C, bevorzugt zwischen 20 und 200 °C durchgeführt. Der Druck liegt dabei zwischen 0,01 und 300 bar, bevorzugt zwischen 0,1 und 125 bar, besonders bevorzugt zwischen 1 und 64 bar.

Bei Hydrierungen in der Füssigphase beträgt die Weight-Hourly-Space-Velocity (WHSV), angegeben in Gramm Substrat pro Gramm Katalysator pro Stunde, bevorzugt 0,01 bis 100 h⁻¹, besonders bevorzugt 0,1 - 50 h⁻¹, ganz besonders bevorzugt 0,5 - 10 h⁻¹. Bei Hydrierungen in der Gasphase beträgt die Weight-Hourly-Space-Velocity (WHSV), angegeben in Gramm Substrat pro Gramm Katalysator pro Stunde, bevorzugt 0,1 bis 200 h⁻¹, besonders bevorzugt 0,5 - 100 h⁻¹, ganz besonders bevorzugt 1 - 50 h⁻¹.

Die Hydrierung in der Flüssigphase, egal ob homogen oder heterogen katalysiert, kann ohne oder in Gegenwart von zusätzlichen Lösemitteln durchgeführt werden. Beispiele für geeignete Lösemittel sind aliphatische und cycloaliphatische Kohlenwasserstoffe wie beispielsweise C₃-C₁₆-Alkane, Mischungen niederer oder höherer Alkane (C₃-C₂₀), Cyclohexan, Cyclooctan und Ethylcyclohexan; Alkohole wie beispielsweise Methanol, Ethanol, Propanol, Isopropanol, n-Butanol, Isobutanol, 2-Ethylhexanol, Isononanol und Isotridecanol; Polyole wie beispielsweise Ethylenglykol, Propylenglykol, 1,3-Propandiol und 1,4-Butandiol; Carbonsäureester wie beispielsweise Essigsäureethylester; Ether wie beispielsweise Dipropylether, Diethylether, Dimethylether, Methyl-tert.-butylether, Methyloctylether, 3-Methoxyoctan, Dioxan, Tetrahydrofuran, Alkylether von Ethylenglykol, Diethylenglykol und Polyethylenglykol; Sulfolan, Dimethylsulfoxid, Ethylencarbonat, Propylencarbonat und Wasser. Die Lösemittel kommen allein oder auch als Mischungen verschiedener Lösemittel zum Einsatz.

Bei der Hydrierung in der Flüssigphase können auch mehrere flüssige Phasen vorliegen. Dieses Verfahren ist besonders vorteilhaft, wenn Katalysator und Produkt in unterschiedlichen Phasen vorliegen, da dann der Katalysator einfach über eine Phasentrennung abgetrennt werden kann. Oftmals bildet dabei Wasser eine der flüssigen Phasen aus. Es werden beispielsweise aber auch perfluorierte Kohlenwasserstoffe, Ionische Flüssigkeiten und überkritisches Kohlendioxid eingesetzt (zu Ionischen Flüssigkeiten vgl. P. Wasserscheid, W. Keim, Angew. Chem., Int. Ed. 2000, 39, 3772 - 3789). Einen Überblick über Wasser als Trägerphase für den Katalysator findet man beispielsweise in B. Cornils, W. A. Herrmann (Eds.) "Aqueous-Phase Organometallic Catalysis", Wiley-VCH, Weinheim, New-York, Chichester, Brisbane, Singapore, Toronto, 1998, Seiten 352-361.

Bei den Hydrierungen können neben Wasserstoff und ggf. Substrat noch andere Gase anwesend sein. Beispielsweise können Stickstoff und/oder Argon, aber auch unter den Hydrierbedingungen gasförmige Alkane wie beispielsweise Methan, Propan oder Butan zugesetzt werden, oder bereits im Hydriergas enthalten sein.

Die Hydrierung im erfindungsgemäßen Verfahren kann kontinuierlich, semi-kontinuierlich oder diskontinuierlich (batchweise) erfolgen. Bevorzugt wird die kontinuierliche Verfahrensführung.
Vorzugsweise wird in der Hydrierung ein möglichst vollständiger Umsatz des Telomers angestrebt. Es ist aber auch möglich, die Reaktion nach einem Teilumsatz abzubrechen und die nicht umgesetzte Menge des Telomers nach Abtrennung von den restlichen Komponenten in die Hydierreaktion zurückzuführen oder gegebenenfalls anderweitig zu verwerten.

Das Hydrierprodukt (hydriertes Telomer) wird zu Olefin und weiteren Spaltprodukten umgesetzt. Hierzu ist es gegebenenfalls sinnvoll, das Produkt nach der Hydrierreaktion durch physikalische Verfahren aufzureinigen. Grundsätzlich können alle Verfahren oder Kombinationen von Verfahren zur Anwendung kommen, mit denen die Nebenprodukte ganz oder teilweise vom hydrierten Telogen abgetrennt werden können. Bevorzugte Trenntechnik ist die Destillation. Für die destillative Trennung können alle verfügbaren Techniken eingesetzt werden, beispielsweise Bodenkolonnen, Kolonnen mit Packungen, Trennwandkolonnen, Extraktivdestillation, Dünnschichtverdampfer und Fallfilmverdampfer. Die destillative Trennung kann in einem oder in mehreren Schritten erfolgen und ist abhängig von den Siedepunkten der im Produktgemisch enthaltenen Komponenten.

Im Spaltprozeß des erfindungsgemäßen Verfahrens wird das hydrierte Telomer gespalten, wobei das gewünschte 1-Olefin entsteht.
Die Spaltung kann sowohl in der Flüssigphase als auch in der Gasphase durchgeführt werden; bevorzugt ist die Spaltung in der Gasphase. Die Spaltung des hydrierten Telomers kann in Gegenwart jeder Menge anderer Stoffe erfolgen, die unter den Spaltbedingungen inert beziehungsweise weitgehend inert sind. Beispielsweise können Stickstoff oder Argon, aber auch Wasser, Wasserdampf oder Alkane wie beispielsweise Methan, Propan oder Butan zugesetzt werden. Bevorzugt liegt der Anteil dieser inerten Stoffe zwischen 0 und 98 Vol.-%, besonders bevorzugt zwischen 0 und 50 Vol.-%.

Die Spaltungsreaktion kann thermisch ohne Katalysator oder katalysiert in Anwesenheit von heterogenen Katalysatoren, jeweils kontinuierlich, semi-kontinuierlich oder diskontinuierlich (batchweise) durchgeführt werden.

Im Spaltprozess des erfindungsgemäßen Verfahrens wird das hydrierte Telomer gespalten, wobei das gewünschte 1-Olefin entsteht. Spaltungsreaktionen dieser Art sind bereits in der Literatur beschrieben. So sind die Spaltungen von Alkoholen und Estern Standardmethoden zur Darstellung von Olefinen (vgl. Houben-Weyl, *Methoden der Organischen Chemie*, Georg Thieme Verlag, Stuttgart, vierte Auflage, Band 5/1b, Seite 45 ff und 105 ff).

JP 02172924 beschreibt die Spaltung von 1-Octanol, erhalten aus einer Telomerisationsreaktion und anschließender Hydrierung, zu 1-Octen. Als Katalysator kommt unter anderem mit Natriumhydroxid modifiziertes Calciumphosphat zum Einsatz.

EP 0 440 995 beschreibt die Spaltung von Alkylestern, erhalten aus einer Telomerisationsreaktion und anschließender Hydrierung, zu 1-Octen. Es werden keine Katalysatoren in der Spaltungsreaktion eingesetzt.

Die Spaltung von Ethern ist ebenfalls bekannt. Mehrere Arbeiten wurden zu Beginn des 20. Jahrhunderts publiziert, beispielsweise die Spaltung von Ethern über saurem Ton (japanese acid clay) (W. Ipatiew, *Berichte der Deutschen Chemischen Gesellschaft,* **1904**, *37*, 2961; K. Kashima, *Bull. Chem. Soc. Jpn.* **1930**, 25).
Die Spaltung eines Methylethers über Alumina, Aluminiumphosphaten, Aluminiumsilikaten und Mischungen von Aluminiumsilikaten mit Metallphosphaten und Metallsulfaten ist Gegenstand des Patents US 2561483.

In WO 92/10450 wird der Einsatz von sauren Katalysatoren bevorzugt, wobei hauptsächlich Aluminiumoxide, die gegebenenfalls modifiziert wurden, zum Einsatz kommen.
In CN 1158277 A werden Katalysatoren, ausgewählt aus modifiziertem SiO₂, Thoriumoxid, den Oxiden der Erdalkalimetalle, der seltenen Erden und der Metalle der Gruppe IV B für die Spaltung von Ethern beansprucht. Die Patentanmeldung CN 1165053 beschreibt den Einsatz von Magnesium-Silizium-Oxiden zur Spaltung von Octylmethylether. Mit diesen Katalysatoren konnten bei Umsätzen des Octylmethylethers von über 80% Selektivitäten zum 1-Octen von über 95% erhalten werden.
Im erfindungsgemäßen Verfahren wird die Spaltung zum 1-Olefin bevorzugt als eine heterogen katalysierte Gasphasenreaktion durchgeführt. Als Katalysatoren können sowohl saure und supersaure Katalysatoren, wie natürliche Tone, Säuren auf Trägermaterialien, saure Metalloxide und Metallsulfide, Metallsalze, Metalloxide, Zeolithe, als auch basische oder stark basische Katalysatoren, wie Basen auf Trägermaterialien, basische Metalloxide, Metallsalze, gemischte Oxide und Zeolithe (meist ausgetauscht mit Alkali oder Erdalkalimetallen) eingesetzt werden.
Beispiele für die oben genannten Katalysatoren finden sich unter anderem in "*New solid acids an bases: their catalytic properties*" by K. Tanabe et al., 1989, Elsevier Science Publishers, Amsterdam, Seiten 1-3.
Bevorzugt eingesetzt werden basische und stark basische Katalysatoren. Besonders bevorzugt werden Alkalimetallhydroxyde/oxide oder Erdalkalimetallhydroxide/oxide optional auf Trägermaterialien wie Silica, Alumina oder Carbonaten eingesetzt. Der Anteil an Metallhydroxiden auf dem Träger beträgt bevorzugt 0,01 Gew.% bis 20 Gew.%, besonders bevorzugt 0,1 Gew.% bis 10 Gew.%. Weiterhin können die zur Spaltung eingesetzten Katalysatoren Alkalimetalloxide, Erdalkalimetalloxide, Zinkoxid, Aluminiumoxid, Yttriumoxid, Lanthanoxid, Ceroxid, Thoriumoxid, Titanoxid, Zirconiumoxid, Zinnoxid, Alkali- und Erdalkalimetallcarbonate, -hydrogencarbonate und -wolframate, Mischoxide von Silizium und/oder Aluminium mit Alkali- und Erdalkalimetallen, Zink, Thorium, Titan, Zirconium, Wolfram, Zinn, Molybdän enthalten. Ebenfalls bevorzugt eingesetzt werden Hydrotalcite.

Die Katalysatoren werden nach bekannten Methoden hergestellt. Gängige Methoden sind beispielsweise Fällung oder Tränkung und anschließende Calcinierung.

Die Spaltung kann bei Temperaturen zwischen 100 und 800 °C, bevorzugt zwischen 200 und 450 °C, besonders bevorzugt zwischen 300 und 350 °C erfolgen. Der Druck (absolut), unter dem die Spaltung durchgeführt wird, liegt typischerweise zwischen 0,1 und 25 bar. Bevorzugt werden Drücke zwischen 0,2 und 5 bar, besonders bevorzugt zwischen 1 und 2 bar. Die Weight-Hourly-Space-Velocity (WHSV), angegeben in Gramm Substrat pro Gramm Katalysator pro Stunde, beträgt bevorzugt 0,01 bis 30 h⁻¹, besonders bevorzugt 0,1 - 15 h⁻¹, ganz besonders bevorzugt 0,5 - 10 h⁻¹.
Die Spaltung kann unter vollständigem oder teilweisen Umsatz durchgeführt werden. Nicht umgesetztes Edukt kann, nach Abtrennung des gebildeten 1-Olefins und gegebenenfalls anderer Spaltungsprodukte, in die Spaltung zurückgeführt werden. Es ist dabei auch möglich, nur das 1-Olefin und gegebenenfalls einen Teil der Spaltprodukte abzutrennen und die Rückführung in die Vorreinigung vor der eigentlichen Spaltung zurückzuführen.

Bevorzugt wird die Spaltung unter teilweisem Umsatz durchgeführt. Der Umsatz beträgt dabei zwischen 10 und 95 %, besonders bevorzugt zwischen 30 und 90 %, ganz besonders bevorzugt zwischen 40 und 95 %.

Die Abtrennung des Zielprodukts, des 1-Olefins, von den anderen Komponenten des Austrags der Spaltung erfolgt nach bekannten Verfahren wie beispielsweise Phasenseparation, Extraktion, Wäsche, Destillation oder Fällung. Bevorzugtes Verfahren ist die Destillation.

Das in der Spaltung erhaltene Nucleophil (z. B. Methanol) kann optional in den Telomerisationsreaktor zurückgeführt werden.
Bei der Herstellung von 1-Octen aus 1,3-Butadien nach dem erfindungsgemäßen Verfahren können neben dem 1-Octen geringe Mengen anderer C₈-Olefine entsehen. So kann 2-Octen durch Isomerisierung des 1-Octens gebildet werden, 3-Octen kann aus dem 2-Octen entstehen usw. Auch Octan und Octadiene können gebildet werden. Zur Erreichung einer sehr hohen 1-Octen-Reinheit (> 97 %) kann es daher notwendig sein, einen Teil dieser C₈-Komponenten abzutrennen. Dies kann über eine destillative Aufreinigung erfolgen. Diese kann sowohl zusammen mit der Abtrennung anderer Produkte aus dem Spaltungsschritt erfolgen, oder separat als Aufreinigung eines zuvor isolierten C₈-Schnitts erfolgen. Die im erfindungsgemäßen Verfahren als Nebenprodukte anfallenden C8-Olefine mit innenständigen Doppelbindungen sind selbst wertvolle Edukte für chemische Prozesse, zum Beispiel können sie in Hydroformylierungsreaktionen eingesetzt werden.

Die nach dem erfindungsgemäßen Verfahren erhaltenen 1-Olefine sind besonders als Comonomer in Polymerisationsreaktionen von Ethen oder Propen geeignet. Ganz besonders hierfür geeignet ist 1-Octen.

Die folgenden Beispiele sollen die Erfindung erläutern, nicht aber ihren Anwendungsbereich beschränken, der sich aus der Beschreibung und den Patentansprüchen ergibt.

### Beispiele 1-3

Telomerisation von 1,3-Butadien mit Methanol.

### Beispiel 1

In einem 3-Liter Autoklav (Firma Büchi) wurden 286 g entgastes Methanol, 562 g 1,3-Butadien, 0.74 g Natrimhydroxid, 50 g Cyclooctan (interner GC Standard) und 0.54 g 4-t-Butylcatechol unter Schutzgas vorgelegt und auf 80 °C erwärmt. 0.0543 g Palladiumacetylacetonat und 0,1208 g 1,3-Bis(2,4,6-timethylphenyl)imidazoliumchlorid wurden separat unter Schutzgas in 47,4 g entgastem Methanol gelöst. Die Reaktion wurde durch Zugabe dieser Lösung (aus einer Druckbürette) in den Autoklaven gestartet und der Reaktionsverlauf durch gaschromatographische Analyse von regelmäßig entnommenen Proben verfolgt. Nach 180 Minuten waren 85 % des Butadiens umgesetzt, nach 420 Minuten mehr als 99 %. Der Versuch wurde durch Herunterkühlen des Autoklaven beendet. Die Selektivität der Reaktion zum 2,7-Octadienyl-1-methylether (1-Methoxy-2,7-octadien, 1-MODE) war nach gaschromatographischer Analyse des Reaktoraustrags > 96 %.

### Beispiel 2

In einem 3-Liter Autoklav (Firma Büchi) wurden 209 g entgastes Methanol, 478 g 1,3-Butadien, 1,36 g Natrimhydroxid, 50 g Cyclooctan (interner GC Standard) und 0,52 g 4-t-Butylcatechol unter Schutzgas vorgelegt und auf 80 °C erwärmt. 0,0500 g Palladiumacetylacetonat und 0,1253 g 1,3-Bis(2,4,6-timethylphenyl)-4,5-dihydroimidazoliumtetrafluroborat wurden separat unter Schutzgas in 51 g entgastem Methanol gelöst. Die Reaktion wurde durch Zugabe dieser Lösung (aus einer Druckbürette) in den Autoklaven gestartet und der Reaktionsverlauf durch gaschromatographische Analyse von regelmäßig entnommenen Proben verfolgt. Nach 105 Minuten waren 85 % des Butadiens umgesetzt, nach 285 Minuten mehr als 98 %. Der Versuch wurde durch Herunterkühlen des Autoklaven beendet. Die Selektivität der Reaktion zum 2,7-Octadienyl-1-methylether war nach gaschromatographischer Analyse des Reaktoraustrags > 96 %.

### Beispiel 3

In einem 3-Liter Autoklav (Firma Büchi) wurden 207 g entgastes Methanol, 521 g 1,3-Butadien, 1,38 g Natrimhydroxid, 50 g Cyclooctan (interner GC Standard) und 0,46 g 4-t-Butylcatechol unter Schutzgas vorgelegt und auf 80 °C erwärmt. 0,0494 g Palladiumacetylacetonat und 0,1382 g 1,3-Bis(2,6-di-i-propylphenyl)imidazoliumchlorid wurden separat unter Schutzgas in 49,1 g entgastem Methanol gelöst. Die Reaktion wurde durch Zugabe dieser Lösung (aus einer Druckbürette) in den Autoklaven gestartet und der Reaktionsverlauf durch gaschromatographische Analyse von regelmäßig entnommenen Proben verfolgt. Nach 210 Minuten waren 85 % des Butadiens umgesetzt, nach 420 Minuten mehr als 98 %. Der Versuch wurde durch Herunterkühlen des Autoklaven beendet. Die Selektivität der Reaktion zum 2,7-Octadienyl-1-methylether betrug nach gaschromatographischer Analyse des Reaktoraustrags 87 %, die zum 1,3,7-Octatrien 4 %. Das Verhältnis 2,7-Octadienyl-1-methylether zu 3-Methoxyoctadien betrug 91.6 : 8.4.

### Beispiel 4

In einem Schlenk-Kolben wurden unter einer Argon Schutzgasatmosphäre 111,1 mg Natriumhydroxid und 1,6 mg des Palladiumkomplexes **I-a** in 17,8 g Methanol gelöst. Die Lösung wurde unter Schutzgas in einen 100 ml Autoklaven (der Firma Parr) überführt, der Autoklav abgekühlt und 15 g 1,3-Butadien einkondensiert. Der Autoklav wurde für 16 Stunden auf 90 °C aufgeheizt. In diesem Zeitraum setzten sich 87 % des 1,3-Butadiens um. Die Selektivität der Reaktion zum 1-Methoxyocta-2,7-dien betrug 95 %, die zum 3-Methoxyocta-1,7-dien 2,4%.

### Beispiel 5

In einem 70 l Stahlautoklaven wurden 6,4 kg Methanol vorgelegt. Im Methanol wurden 35 g Natriumhydroxid gelöst, zu dieser Lösung 1,9 g 4-t-Butylcatechol, 1,8 g Palladium(II)acetat und 6 g 1,3-Bis(2,4,6-timethylphenyl)imidazoliumchlorid gegeben. Nach Zugabe von 15 kg 1,3-Butadien wurde der Autoklav erwärmt. Nach dem Starten der exothermen Reaktion (bei ca. 70 °C) stieg die Innentemperatur auf ein Maximum von 125 °C, danach kühlte sich die Reaktionsmischung wieder ab und wurde über die Heizung bei konstant 80 °C gehalten. Nach sechs Stunden wurde der Reaktor auf Raumtemperatur abgekühlt. Laut GC Analyse wurden 95 % des 1,3-Butadiens umgesetzt, die Selektivität zum 1-Methoxyocta-2,7-dien betrug 93,0 %, die zum 3-Methoxyocta-1,7-dien 2.9%.

### Beispiele 6- 9

### Hydrierung der Telomere.

Die Reaktionsgemische wurden gaschromatisch auf einer FFAP-Säule der Fa. Hewlett-Packard analysiert. Als Hydrierkatalysator wurde der handelsübliche Katalysator H 14184r, hergestellt von der Degussa AG, eingesetzt.
Seine Eigenschaften sind durch den Hersteller wie folgt beschrieben:

| | |
|---|---|
| Pd-Anteil | 0,5 Gew.-% |
| Träger | Al₂O₃ |
| Form | Extrudate |
| Durchmesser | 1,2 mm |
| Länge | 2 - 8 mm |
| Schüttdichte | 600 kg/m³ |
| BET-Oberfläche | 200 m²/g |
| Spez. Porenvolumen | 0.65 cm³/g |
| Reduktionvorschrift | keine, Katalysator ist vorreduziert |

### Beispiel 6

50 g des Katalysators wurden in dem Katalysatorkörbchen eines 1000 ml-Druckreaktors vorgelegt und mit 492 g flüssigem 1-Methoxy-2,7-octadien (1-MODE) versetzt. Das trans/cis-Verhältnis im 1-MODE betrug 0.94. Die Hydrierung des 1-MODE erfolgte mit reinem Wasserstoff bei einem Druck von 20 bar und einer Temperatur von 40 °C. Die Hydrierung war nach 10 h abgeschlossen. Der Umsatz an 1-MODE betrug danach 99.9 %. Die Ausbeute an 1-Methoxyoctan (1-MOAN) lag bei 99.9 %.

### Beispiel 7

50 g des Katalysators wurden in dem Katalysatorkörbchen eines 1000 ml-Druckreaktors vorgelegt und mit 492 g flüssigem 1-Methoxy-2,7-octadien (1-MODE) versetzt. Das trans/cis-Verhältnis im 1-MODE betrug 0,94. Die Hydrierung des 1-MODE erfolgte mit reinem Wasserstoff bei einem Druck von 20 bar und einer Temperatur von 60 °C. Die Hydrierung war nach 6 h abgeschlossen. Der Umsatz an 1-MODE betrug danach 99,9 %. Die Ausbeute an 1-Methoxyoctan (1-MOAN) lag bei 99,9 %.

### Beispiel 8

50 g des Katalysators wurden in dem Katalysatorkörbchen eines 1000 ml-Druckreaktors vorgelegt und mit 492 g flüssigem 1-Methoxy-2,7-octadien (1-MODE) versetzt. Das trans/cis-Verhältnis im 1-MODE betrug 0,94. Die Hydrierung des 1-MODE erfolgte mit reinem Wasserstoff bei einem Druck von 30 bar und einer Temperatur von 40 °C. Die Hydrierung war nach 5,5 h abgeschlossen. Der Umsatz an 1-MODE betrug danach 99,9 %. Die Ausbeute an 1-Methoxyoctan (1-MOAN) lag bei 99,9 %.

### Beispiel 9

50 g des Katalysators wurden in dem Katalysatorkörbchen eines 1000 ml-Druckreaktors vorgelegt und mit 492 g flüssigem 1-Methoxy-2,7-octadien (1-MODE) versetzt. Das trans/cis-Verhältnis im 1-MODE betrug 0,94. Die Hydrierung des 1-MODE erfolgte mit reinem Wasserstoff bei einem Druck von 30 bar und einer Temperatur von 60 °C. Die Hydrierung war nach 4 h abgeschlossen. Der Umsatz an 1-MODE betrug danach 99,9 %. Die Ausbeute an 1-Methoxyoctan (1-MOAN) lag bei 99.9 %.

### Beispiel 10 - Spaltung an einem sauren Katalysator

Das Produkt der Hydrierung 1-Methoxyoctan (1-MOAN, Methyl-n-octylether) wurde mit einer Reinheit von rd. 98 Gew.-% (2% Hochsieder) für die Spaltung in einem Durchfluß-Festtbettreaktor in Gegenwart eines Silica-Alumina-Katalysators eingesetzt. Beim Katalysator handelt es sich um einen handelsüblichen Katalysator mit der Bezeichnung K306 der Süd-Chemie AG.
Vor dem Eintritt in den Reaktor wurde das flüssige Edukt in einem vorgeschalteten Verdampfer bei 220 °C verdampft. Bei einer Reaktionstemperatur von 250 °C im Reaktor wurden stündlich 7,7 g/h Edukt durch 10 g Katalysator in Granulatform in der Gasphase, entsprechend einem WHSV-Wert von 0,77 h⁻¹, durchgeleitet. Das gasförmige Produkt wurde in einem Kühler abgekühlt und in flüssiger Form in einer Glasvorlage gesammelt.
Die GC-Analyse des Spaltungsproduktes ist in Tabelle 1 wiedergegeben.

**Tabelle 1**

| Spaltung von 1-MOAN am Silica-Alumina-Katalysator K306 | |
|---|---|
| Komponente | Produkte der MOAN-Spaltung (Gew-%) |
| 1-Octen | 2,7 |
| t-4-Octen | 2,0 |
| t-3-Octen/c-4-Octen | 5,3 |
| c-3-Octen | 1,6 |
| t-2-Octen | 6,2 |
| c-2-Octen | 3,8 |
| Methanol | 6,2 |
| 1-MOAN | 67,1 |
| Rest | 5,1 |

Wie man aus der Tabelle 1 entnehmen kann, wird das 1-MOAN mit einer relativ geringen 1-Octen-Selektivität (≈ 8,7 %) zum gewünschten Wertprodukt 1-Octen gespalten.

### Beispiel 11

Das Produkt der Hydrierung 1-Methoxyoctan (1-MOAN, Methyl-n-octylether) wurde mit einer Reinheit von rd. 98 Gew.-% (2% Hochsieder) für die Spaltung in einem Durchfluß-Festtbettreaktor in Gegenwart eines mit Natronlauge modifizerten Aluminiumoxids (Al₂O₃ mit 1 Gew.-% Na₂O) eingesetzt.
Vor dem Eintritt in den Reaktor wurde das flüssige Edukt in einem vorgeschalteten Verdampfer bei 220 °C verdampft. Bei einer Reaktionstemperatur von 350 °C im Reaktor wurden stündlich 20 g Edukt durch 18 g Katalysator in Kugelform in der Gasphase, entsprechend einem WHSV-Wert von 1,1 h⁻¹ , durchgeleitet. Das gasförmige Produkt wurde in einem Kühler abgekühlt und in flüssiger Form in einer Glasvorlage gesammelt.
Die GC-Analyse des Spaltungsproduktes ist in Tabelle 2 wiedergegeben.

**TABELLE 2**

| Spaltung von 1-MOAN am Na-modifizierten Al₂O₃ -Katalysator | |
|---|---|
| Komponente | Podukte der 2-Octanol-Spaltung (Gew-%) |
| 1-Octen | 32,95 |
| t-4-Octen | 0,02 |
| t-3-Octen/c-4-Octen | 0,01 |
| c-3-Octen | 0,01 |
| t-2-Octen | 0,77 |
| c-2-Octen | 1,25 |
| Methanol | 9,16 |
| MOAN | 47,38 |
| Rest | 8,47 |

Wie man aus der Tabelle 2 entnehmen kann, wird das 1-MOAN mit einer hohen 1-Octen-Selektivität (> 92 % ) zum gewünschten Wertprodukt 1-Octen gespalten.
Die unter Rest aufgeführten Nebenprodukte enthalten Komponenten, die ebenfalls zum 1-Octen gespalten werden können, unter anderem Dioctylether. Auch diese können ggf. in die Spaltung zurückgeführt werden.

## Patentansprüche

1. Verfahren zur Herstellung von 1-Olefinen mit 8 bis 16 Kohlenstoffatomen durch Telomerisation eines Ausgangsolefins mit mindestens zwei konjugierten Doppelbindungen mit einem Nucleophil unter Anwesenheit eines Palladiumkatalysators, Hydrierung des so erhaltenen Telomers und anschließende Spaltung zum 1-Olefin,
**dadurch gekennzeichnet,**
**dass** als Palladiumkatalysator ein Palladiumcarbenkomplex verwendet wird.

2. Verfahren nach Anspruch 1,
**dadurch gekennzeichnet,**
**dass** die Carbenliganden das Strukturelement 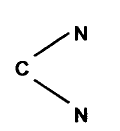 aufweisen, worin C das Carbenkohlenstoffatom darstellt, das an das Palladiumatom bindet.

3. Verfahren nach Anspruch 1,
**dadurch gekennzeichnet,**
**dass** als Carbenligand eine oder mehrere Verbindungen der Formeln I oder II verwendet werden, 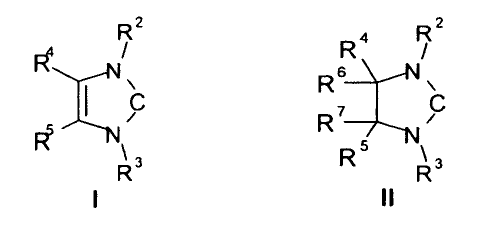 wobei R² und R³ unabhängig voneinander eine lineare, verzweigte oder cyclische C₁ bis C₂₄-Alkylgruppe oder eine C₅ bis C₁₈-Arylgruppe sind, wobei die Alkylgruppe und die Arylgruppe unabhängig voneinander die Substituenten -CN, -COOH, -COO-Alkyl-(C₁-C₈), -CO-Alkyl-(C₁-C₈), -Aryl-(C₆-C₁₈), -Alkyl-(C₁-C₂₄), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, - NH₂, -F, -Cl, -OH, -CF₃, -NO₂, Ferrocenyl enthalten können, und wobei R⁴ bis R⁷ unabhängig voneinander Wasserstoff, -CN, -COOH, -COO-Alkyl-(C₁-C₈), -CO-Alkyl-(C₁-C₈), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ oder eine lineare, verzweigte oder cyclische C₁ bis C₂₄-Alkylgruppe oder C₆ bis C₁₈-Arylgruppe ist und die Alkylgruppe und Arylgruppe unabhängig voneinander die Substituenten -CN, -COOH, -COO-Alkyl-(C₁-C₈), -CO-Alkyl-(C₁-C₈), -Aryl-(C₆-C₁₀), -Alkyl(C₁-C₂₄), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂, enthalten kann, und wobei die Reste R⁴ und R⁵ auch Teil eines verbrückenden aliphatischen oder aromatischen Ringes sein können.

4. Verfahren nach einem der Ansprüche 1 bis 3,
**dadurch gekennzeichnet,**
**dass** als Nucleophile Verbindungen der Formel III, IV oder V eingesetzt werden, 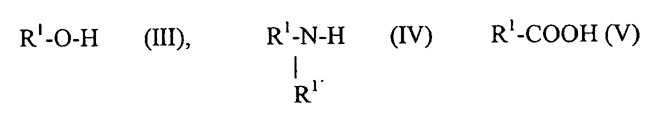 worin R¹, R^{1'} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer Carboxylgruppe oder einer C₅ bis C₁₈ Arylgruppe, wobei diese Gruppen Substituenten ausgewählt aus der Gruppe -CN, -COOH, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ enthalten können, und wobei die Reste R¹, R^{1'} über kovalente Bindungen miteinander verknüpft sein können.

5. Verfahren nach einem der Ansprüche 1 bis 4,
**dadurch gekennzeichnet,**
**dass** als Nucleophil Methanol, Ethanol, 2-Ethlyhexanol, Octanol, Octenol, Octadienol, Isopropanol, n-Propanol, Isobutanol, n-Butanol, Isononanol, Ameisensäure, Essigsäure, Propionsäure, n-Butansäure, iso-Butansäure, Benzoesäure, Phthalsäure und/oder Wasser eingesetzt wird.

6. Verfahren nach einem der Ansprüche 1 bis 5,
**dadurch gekennzeichnet,**
**dass** die Telomerisation nur bis zu einem Umsatz des Ausgangsolefins von maximal 95 % durchgeführt wird.

7. Verfahren nach einem der Ansprüche 1 bis 6,
**dadurch gekennzeichnet,**
**dass** zur Hydrierung des Telomers ein heterogener oder homogener Katalysator eingesetzt wird.

8. Verfahren nach einem der Ansprüche 1 bis 7,
**dadurch gekennzeichnet,**
**dass** zur Hydrierung ein heterogener Katalysator, enthaltend mindestens ein Metall der gruppen 6-11 des Periodensystems der Elemente eingesetzt wird.

9. Verfahren nach einem der Ansprüche 1 bis 8,
**dadurch gekennzeichnet,**
**dass** zur Spaltung des hydrierten Telomers ein heterogener Katalysator eingesetzt wird.

10. Verfahren nach einem der Ansprüche 1 bis 9,
**dadurch gekennzeichnet,**
**dass** zur Spaltung des hydrierten Telomers ein basischer oder stark basischer oder ein sauer oder stark saurer Katalysator eingesetzt wird.

11. Verfahren nach einem der Ansprüche 1 bis 10,
**dadurch gekennzeichnet,**
**dass** zur Spaltung des hydrierten Telomers Alkalimetallhydroxyde/oxide oder Erdalkalimetallhydroxide/oxide enthaltende Katalysatoren eingesetzt werden.

12. Verfahren nach einem der Ansprüche 1 bis 10,
**dadurch gekennzeichnet,**
**dass** zur Spaltung des hydrierten Telomers Alkalimetalloxide, Erdalkalimetalloxide, Zinkoxid, Aluminiumoxid, Yttriumoxid, Lanthanoxid, Ceroxid, Thoriumoxid, Titanoxid, Zirconiumoxid, Zinnoxid, Alkali- und Erdalkalimetallcarbonate, -hydrogencarbonate oder -wolframate enthaltende Katalysatoren eingesetzt werden.

13. Verfahren nach einem der Ansprüche 1 bis 10,
**dadurch gekennzeichnet,**
**dass** zur Spaltung des hydrierten Telomers Hydrotalzite, Mischoxide von Silizium und/oder Aluminium mit Alkali- und Erdalkalimetallen, Zink, Thorium, Titan, Zirconium, Wolfram, Zinn oder Molybdän enthaltende Katalysatoren eingesetzt werden.

14. Verfahren nach einem der Ansprüche 1 bis 13,
**dadurch gekennzeichnet,**
**dass** die Spaltung des hydrierten Telomers in der Gasphase durchgeführt wird.

15. Verfahren nach einem der Ansprüche 1 bis 14,
**dadurch gekennzeichnet,**
**dass** die Spaltung des hydrierten Telomers bei Temperaturen zwischen 100 und 800 °C durchgeführt wird.

16. Verfahren nach einem der Ansprüche 1 bis 15,
**dadurch gekennzeichnet,**
**dass** die Spaltung bis zu einem Umsatz des hydrierten Telomers von 10-95 % durchgeführt wird.

17. Verfahren nach einem der Ansprüche 1 bis 16,
**dadurch gekennzeichnet,**
**dass** als Ausgangsolefin mit mindestens zwei konjugierten Doppelbindungen 1,3-Butadien oder Isopren eingesetzt wird.

18. Verfahren nach Anspruch 17,
**dadurch gekennzeichnet,**
**dass** das Ausgangsolefin in Mischung mit anderen Kohlenwasserstoffen eingesetzt wird.

19. Verfahren nach den Ansprüchen 1 bis 18,
**dadurch gekennzeichnet,**
**dass** die Telomerisation bei Temperaturen von 10 bis 180 °C und einem Druck von 1 bis 300 bar durchgeführt wird.

20. Verfahren nach den Ansprüchen 1 bis 19,
**dadurch gekennzeichnet,**
**dass** das Verhältnis von Carbenliganden zu Pd [Mol/Mol] 0,01:1 bis 250:1 beträgt.

21. Verfahren nach den Ansprüchen 1 bis 20,
**dadurch gekennzeichnet,**
**dass** die Palladiumcarbenkomplexe als solche in der Telomerisationsreaktion eingesetzt werden.

22. Verfahren nach den Ansprüchen 1 bis 20,
**dadurch gekennzeichnet,**
**dass** die Palladiumcarbenkomplexe während der Telomerisationsreaktion in situ erzeugt werden.

23. Verfahren nach einem der Ansprüche 1 bis 20,
**dadurch gekennzeichnet,**
**dass** die Carbenliganden während der Telomerisationsreaktion in situ erzeugt werden.

24. Verfahren nach den Ansprüchen 1 bis 13,
**dadurch gekennzeichnet,**
**dass** der Telomerisationsreaktion eine basische Komponente mit einem pK_{b}-Wert < 7 zugesetzt wird.

25. Verfahren nach den Ansprüchen 1 bis 23,
**dadurch gekennzeichnet,**
**dass** die Palladiumkonzentration in der Reaktionsmischung der Telomerisation 0,01 bis 1000 ppm beträgt.

## Claims

1. A process for preparing a 1-olefin having from 8 to 16 carbon atoms by telomerization of a starting olefin having at least two conjugated double bonds with a nucleophile in the presence of a palladium catalyst, hydrogenation of the telomer obtained in this way and subsequent cleavage to the 1-olefin,
**characterized in that** the palladium catalyst used is a palladium-carbene complex.

2. A process according to claim 1, **characterized in that** the carbene ligand comprises the structural element 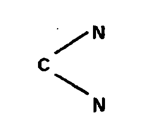 where C is the carbene carbon which is bound to the palladium atom.

3. A process according to claim 1, **characterized in that** one or more compounds of the formula I or II 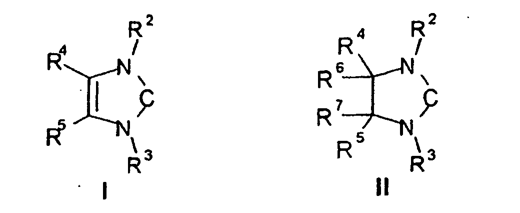 where R² and R³ are each, independently of one another, a linear, branched or cyclic C₁-C₂₄-alkyl group or a C₅-C₁₈-aryl group, where the alkyl group and the aryl group may bear, independently of one another, the substituents -CN, -COOH, -COO-alkyl-(C₁-C₈), -CO-alkyl-(C₁-C₈), -aryl-(C₆-C₁₈), -alkyl-(C₁-C₂₄), -COO-aryl-(C₆-C₁₀), -CO-aryl-(C₆-C₁₀), -O-alkyl-(C₁-C₈), -O-CO-alkyl-(C₁-C₈), -N-alkyl₂-(C₁-C₈), -CHO, -SO₃H, -NH₂, -F, -Cl, -OH, -CF₃, -NO₂, ferrocenyl, and R⁴ to R⁷ are each, independently of one another, hydrogen, -CN, -COOH, -COO-alkyl-(C₁-C₈), -CO-alkyl- (C₁-C₈), -COO-aryl-(C₆-C₁₀), -CO-aryl-(C₆-C₁₀), -O-alkyl-(C₁-C₈), -O-CO-alkyl-(C₁-C₈), -N-alkyl₂-(C₁-C₈), -CHO, -SO₃H, -NH₂, -F, -Cl, -OH, -CF₃, -NO₂ or a linear, branched or cyclic C₁-C₂₄-alkyl group or a C₆-C₁₈-aryl group and the alkyl group and aryl group may bear, independently of one another, the substituents -CN, -COOH, -COO-alkyl-(C₁-C₈), -CO-alkyl-(C₁-C₈), -aryl-(C₆-C₁₀), -alkyl-(C₁-C₂₄), -COO-aryl-(C₆-C₁₀), -CO-aryl-(C₆-C₁₀), -O-alkyl-(C₁-C₈), -O-CO-alkyl-(C₁-C₈), -N-alkyl₂-(C₁-C₈), -CHO, -SO₃H, -NH₂, -F, -Cl, -OH, -CF₃, -NO₂, and the radicals R⁴ and R⁵ may also be part of a bridging aliphatic or aromatic ring, are used as carbene ligand.

4. A process according to any of claims 1 to 3,
**characterized in that** a compound of the formula III, IV or V 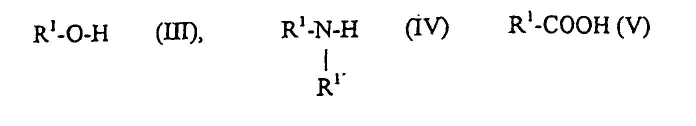 where R¹ and R^{1'} are selected independently from among hydrogen, a linear, branched or cyclic C₁-C₂₂-alkyl group, an alkenyl group, an alkynyl group, a carboxyl group or a C₅-C₁₈-aryl group, which groups may bear a substituent selected from the group consisting of -CN, -COOH, -COO-alkyl-(C₁-C₈), -CO-alkyl-(C₁-C₈), -aryl-(C₅-C₁₀), -COO-aryl-(C₆-C₁₀), -CO-aryl- (C₆-C₁₀), -O-alkyl-(C₁-C₈), -O-CO-alkyl-(C₁-C₈), -N-alkyl₂-(C₁-C₈), -CHO, -SO₃H, -NH₂, -F, -Cl, -OH, -CF₃, -NO₂, and the radicals R¹, R^{1'} may be linked to one another via a covalent bond, is used as nucleophile.

5. A process according to any of claims 1 to 4, **characterized in that** methanol, ethanol, 2-ethylhexanol, octanol, octenol, octadienol, isopropanol, n-propanol, isobutanol, n-butanol, isononanol, formic acid, acetic acid, propionic acid, n-butanoic acid, isobutanoic acid, benzoic acid, phthalic acid and/or water are or is used as nucleophile.

6. A process according to any of claims 1 to 5, **characterized in that** the telomerization is carried out only to a conversion of the starting olefin of not more than 95%.

7. A process according to any of claims 1 to 6, **characterized in that** a heterogeneous or homogeneous catalyst is used for the hydrogenation of the telomer.

8. A process according to any of claims 1 to 7, **characterized in that** a heterogeneous catalyst comprising at least one metal of groups 6-11 of the Periodic Table of the Elements is used for the hydrogenation.

9. A process according to any of claims 1 to 8, **characterized in that** a heterogeneous catalyst is used for the cleavage of the hydrogenated telomer.

10. A process according to any of claims 1 to 9, **characterized in that** a basic or strongly basic or an acidic or strongly acidic catalyst is used for the cleavage of the hydrogenated telomer.

11. A process according to any of claims 1 to 10, **characterized in that** a catalyst comprising an alkali metal hydroxide/oxide or an alkaline earth metal hydroxide/oxide is used for the cleavage of the hydrogenated telomer.

12. A process according to any of claims 1 to 10, **characterized in that** a catalyst comprising an alkali metal oxide, an alkaline earth metal oxide, zinc oxide, aluminium oxide, yttrium oxide, lanthanum oxide, cerium oxide, thorium oxide, titanium oxide, zirconium oxide, tin oxide, an alkali metal or alkaline earth metal carbonate, hydrogencarbonate or tungstate is used for the cleavage of the hydrogenated telomer.

13. A process according to any of claims 1 to 10, **characterized in that** a catalyst comprising a hydrotalcite, a mixed oxide of silicon and/or aluminium with an alkali metal or alkaline earth metal, zinc, thorium, titanium, zirconium, tungsten, tin or molybdenum is used for the cleavage of the hydrogenated telomer.

14. A process according to any of claims 1 to 13, **characterized in that** the cleavage of the hydrogenated telomer is carried out in the gas phase.

15. A process according to any of claims 1 to 14, **characterized in that** the cleavage of the hydrogenated telomer is carried out at a temperature in the range from 100 to 800°C.

16. A process according to any of claims 1 to 15, **characterized in that** the cleavage is carried out to a conversion of the hydrogenated telomer of 10-95%.

17. A process according to any of claims 1 to 16, **characterized in that** 1,3-butadiene or isoprene is used as starting olefin having at least two conjugated double bonds.

18. A process according to claim 17, **characterized in that** the starting olefin is used in admixture with another hydrocarbon.

19. A process according to any of claims 1 to 18, **characterized in that** the telomerization is carried out at a temperature of from 10 to 180°C and a pressure of from 1 to 300 bar.

20. A process according to any of claims 1 to 19, **characterized in that** the ratio of carbene ligand to Pd [mol/mol] is from 0.01:1 to 250:1.

21. A process according to any of claims 1 to 20, **characterized in that** the palladium-carbene complex is introduced as such into the telomerization reaction.

22. A process according to any of claims 1 to 20, **characterized in that** the palladium-carbene complex is generated in situ during the telomerization reaction.

23. A process according to any of claims 1 to 20, **characterized in that** the carbene ligand is generated in situ during the telomerization reaction.

24. A process according to any of claims 1 to 13, **characterized in that** a basic component having a pK_{b} of < 7 is added to the telomerization reaction.

25. A process according to any of claims 1 to 23, **characterized in that** the palladium concentration in the reaction mixture of the telomerization is from 0.01 to 1000 ppm.

## Revendications

1. Procédé de production de 1-oléfines portant 8 à 16 atomes de carbone par télomérisation d'une oléfine de départ, comportant au moins deux doubles liaisons conjuguées, avec un nucléophile en présence d'un catalyseur à base de palladium, hydrogénation du télomère ainsi obtenu et clivage subséquent en 1-oléfine,
**caractérisé en ce que**
comme catalyseur à base de palladium on utilise un complexe de palladium-carbène.

2. Procédé selon la revendication 1,
**caractérisé en ce que**
les ligands de carbène présentent l'élément structurel 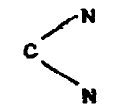 dans lequel C représente l'atome de carbone du carbène qui se lie à l'atome de palladium.

3. Procédé selon la revendication 1,
**caractérisé en ce que**
comme ligand de carbène, on utilise un ou plusieurs composés des formules I ou II, 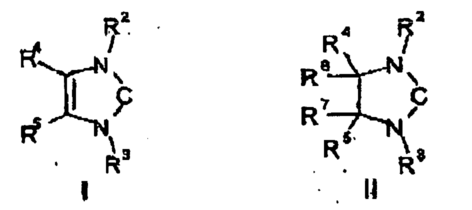 dans lesquelles R² et R³, indépendamment l'un de l'autre, représentent un groupe alkyle linéaire, ramifié ou cyclique en C₁ à C₄ ou un groupe aryle en C₅ à C₁₈, le groupe alkyle et le groupe aryle, indépendamment l'un de l'autre, peuvent contenir les substituants -CN, -COOH, -COO-alkyle-(C₁-C₈), -CO-alkyle-(C₁-C₈), -aryle-(C₆-C₁₈), -alkyle-(C₁-C₂₄), -COO-aryle-(C₆-C₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H, -NH₂, -F, -Cl, -OH, -CF₃, -NO₂, ferrocényle et dans lesquelles R⁴ à R⁷, indépendamment l'un de l'autre, représentent un atome d'hydrogène, -CN, -COOH, -COO-alkyle-(C₁-C₈), -CO-alkyle-(C₁-C₈), -COO-aryle-(C₆-C₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H, -NH₂, -F, -Cl, -OH, -CF₃, -NO₂, ou un groupe linéaire, ramifié ou cyclique alkyle en C₁ à C₂₄ ou aryle en C₆ à C₁₈, et les groupes alkyle et aryle, indépendamment l'un de l'autre, peuvent contenir les substituants -CN, -COOH, -COO-alkyle-(C₁-C₈), -CO-alkyle-(C₁-C₈), -aryle-(C₆-C₁₀), -alkyle-(C₁-C₂₄), -COO-aryle-(C₆-C₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H, -NH₂, -F, -Cl, -OH, -CF₃, -NO₂, et dans lesquelles les radicaux R⁴ et R⁵ peuvent aussi être une partie du cycle aliphatique ou aromatique pontant.

4. Procédé selon l'une quelconque des revendications 1 à 3,
**caractérisé en ce qu'**
on utilise, comme nucléophile, des composés de formule III, IV ou V 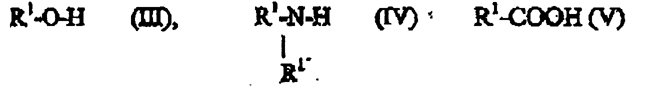 dans lesquelles R¹ et R^{1'}, indépendamment l'un de l'autre, sont choisis parmi l'hydrogène, un groupe alkyle linéaire, ramifié ou cyclique en C₁ à C₂₂, un groupe alcényle, un groupe alcynyle, un groupe carboxyle ou un groupe aryle en C₅ à C₁₈, ces groupes pouvant contenir des substituants choisis dans le groupe -CN, -COOH, -COO-alkyle-(C₁-C₈), -CO-alkyle-(C₁-C₈), -aryle-(C₅-C₁₀), -COO-aryle-(C₆-C₁₀), -CO-aryle-(C₆-C₁₀), -O-alkyle-(C₁-C₈), -O-CO-alkyle-(C₁-C₈), -N-alkyle₂-(C₁-C₈), -CHO, -SO₃H, - NH₂, -F, -Cl, -OH, -CF₃, -NO₂, et dans lesquelles les radicaux R¹ et R^{1'} peuvent être associés ensemble par des liaisons covalentes.

5. Procédé selon l'une quelconque des revendications 1 à 4,
**caractérisé en ce qu'**
on utilise comme nucléophile, du méthanol, de l'éthanol, du 2-éthylhexanol, de l'octanol, de l'octénol, de l'octadiénol, de l'isopropanol, du n-propanol, de l'isobutanol, du n-butanol, de l'isononanol, de l'acide formique, de l'acide acétique, de l'acide propionique, de l'acide n-butanoïque, de l'acide iso-butanoïque, de l'acide benzoïque, de l'acide phtalique et/ou de l'eau.

6. Procédé selon l'une quelconque des revendications 1 à 5,
**caractérisé en ce que**
la télomérisation n'est réalisée que jusqu'à une conversion de l'oléfine de départ d'au maximum 95 %.

7. Procédé selon l'une quelconque des revendications 1 à 6,
**caractérisé en ce qu'**
on utilise un catalyseur hétérogène ou homogène pour hydrogéner le télomère.

8. Procédé selon l'une quelconque des revendications 1 à 7,
**caractérisé en ce que**
pour l'hydrogénation, on utilise un catalyseur hétérogène contenant au moins un métal des groupes 6-11 de la Classification périodique des éléments.

9. Procédé selon l'une quelconque des revendications 1 à 8,
**caractérisé en ce que**
pour le clivage du télomère hydrogéné, on utilise un catalyseur hétérogène.

10. Procédé selon l'une quelconque des revendications 1 à 9,
**caractérisé en ce que**
pour le clivage du télomère hydrogéné, on utilise un catalyseur basique ou fortement basique ou un catalyseur acide ou fortement acide.

11. Procédé selon l'une quelconque des revendications 1 à 10,
**caractérisé en ce que**
pour le clivage du télomère hydrogéné, on utilise des catalyseurs contenant des hydroxydes/oxydes de métaux alcalins ou des hydroxydes/oxydes de métaux alcalino-terreux.

12. Procédé selon l'une quelconque des revendications 1 à 10,
**caractérisé en ce que**
pour le clivage du télomère hydrogéné, on utilise des catalyseurs contenant des oxydes de métaux alcalins, des oxydes de métaux alcalino-terreux, de l'oxyde de zinc, de l'oxyde d'aluminium, de l'oxyde d'yttrium, de l'oxyde de lanthane, de l'oxyde de cérium, de l'oxyde de thorium, de l'oxyde de titane, de l'oxyde de zirconium, de l'oxyde d'étain, des carbonates, hydmgénocarbonates ou wolframates de métaux alcalins et alcalino-terreux.

13. Procédé selon l'une quelconque des revendications 1 à 10,
**caractérisé en ce que**
pour le clivage du télomère hydrogéné, on utilise des catalyseurs contenant des hydrotalcites, des oxydes mixtes de silicium et/ou d'aluminium avec des métaux alcalins ou alcalino-terreux, du zinc, du thorium, du titane, du zirconium, du tungstène, de l'étain ou du molybdène.

14. Procédé selon l'une quelconque des revendications 1 à 13,
**caractérisé en ce que**
le clivage du télomère hydrogéné est réalisé en phase gazeuse.

15. Procédé selon l'une quelconque des revendications 1 à 14,
**caractérisé en ce que**
le clivage du télomère hydrogéné est réalisé à des températures comprises entre 100 et 800°C.

16. Procédé selon l'une quelconque des revendications 1 à 15,
**caractérisé en ce que**
le clivage est réalisé jusqu'à une conversion du télomère hydrogéné de 10 à 95%.

17. Procédé selon l'une quelconque des revendications 1 à 16,
**caractérisé en ce qu'**
on utilise du 1,3-butadiène ou de l'isoprène en tant qu'oléfine de départ comportant au moins deux doubles liaisons conjuguées.

18. Procédé selon la revendication 17,
**caractérisé en ce qu'**
on utilise l'oléfine de départ en mélange avec d'autres hydrocarbures.

19. Procédé selon les revendications 1 à 18,
**caractérisé en ce qu'**
on réalise la télomérisation à des températures de 10 à 180°C et à une pression de 1 à 300 bars.

20. Procédé selon les revendications 1 à 19,
**caractérisé en ce que**
le rapport entre les ligands de carbène et le Pd [mole/mole] est de 0,01:1 à 250:1.

21. Procédé selon les revendications 1 à 20,
**caractérisé en ce que**
les complexes de palladium-carbène sont utilisés en tant que tels dans la réaction de télomérisation.

22. Procédé selon les revendications 1 à 20,
**caractérisé en ce que**
les complexes palladium-carbène sont produits in situ pendant la réaction de télomérisation.

23. Procédé selon l'une quelconque des revendications 1 à 20,
**caractérisé en ce que**
les ligands de carbène sont produits in situ pendant la réaction de télomérisation.

24. Procédé selon les revendications 1 à 13,
**caractérisé en ce qu'**
on ajoute, à la réaction de télomérisation, un composant basique d'une valeur pK_{b} < 7.

25. Procédé selon les revendications 1 à 23,
**caractérisé en ce que**
la concentration en palladium dans le mélange réactionnel de la télomérisation est égal à 0,01 à 1 000 ppm.
